# EUROPEAN PATENT SPECIFICATION

(11) **EP 1 202 762 B1**
(45) Date of publication and mention of the grant of the patent: **06.07.2011**
(21) Application number: 00908665.3
(22) Date of filing: 16.02.2000
(51) Int. Cl.: A61M 5/30

(54) **NEEDLE-LESS HYPODERMIC JET INJECTION DEVICE**
NADELLOSE, SUBKUTANE STRAHLINJEKTIONSVORRICHTUNG
DISPOSITIF D'INJECTION HYPODERMIQUE PAR JET SANS AIGUILLE

(30) Priority: 18.02.1999 US 252131
(43) Date of publication of application: 08.05.2002
(60) Divisional application: 09004095.7
(73) Proprietor: Bioject, Inc., Portland, OR 97224 (US)
(72) Inventor: LANDAU, Sergio, Laguna Nigel, CA 92677 (US)
(74) Representative: Hallam, Arnold Vincent
(86) International application number: PCT/US2000/003938
(87) International publication number: WO 2000/048654

(56) References cited:
- US-A- 2 680 439
- US-A- 3 688 765
- US-A- 4 652 261
- US-A- 4 790 824
- US-A- 4 941 880
- US-A- 5 865 795

## Description

### Cross Reference to Related Application

This application is a Continuation-in-Part of United States Patent Application, Serial No. 09/195,334, filed 18 November 1998, and of United States Patent Application, Serial No. 09/252,131, filed 18 February 1999.

### Background of the Invention

### Field of the Invention

The present invention relates generally to a single-use disposable needle-less (or needle-free) hypodermic jet injection device. Particularly, this invention relates to such a jet injection device which comprises a hand-held injector having a pre-filled drug cartridge sealingly carrying injectable medication, a sealed cylinder of pressurized gas, a pre-energized discharge mechanism for penetrating the gas cylinder, and a trigger device for releasing the discharge mechanism. Features are provided which simultaneously unseal the drug cartridge and prepare the device for performing a jet injection when a user of the device changes it from a storage configuration to a use configuration. When the user actuated the injection device, the trigger device releases the discharge mechanism to penetrate the gas cylinder, which drives a piston of the drug cartridge to effect a hypodermic jet injection.

### Related Technology

Needle-less or needle-free hypodermic jet injection devices have been in commercial use for over 40 years. A number of these devices have used pressurized gas to power a hypodermic jet injection. The related technology includes a number of teachings for gas-powered injection devices, including: U.S. Patent No. 4,596,556, issued 24 June 1986 to J. Thomas Morrow, et al.; U.S. Patent No. 4,913,699; issued 3 April 1990 to James S. Parsons; and U.S. Patent No. 5,730,723, issued 24 March 1998, to Thomas P. Castellano, et al. WIPO publication WO 97/37705 also discloses a gas powered disposable needle-less hypodermic jet injector.

The Morrow, et. al. '556 patent is believed to teach a reusable hypodermic jet injection device in which a housing receives a shell or cartridge having a bore leading to a discharge aperture. Within the bore is received both a plunger sealingly engaging the bore, and a pressurized gas cylinder which rests against the plunger. The injection device includes a ram which has a penetrating tip confronting a penetrable wall section and seal of the gas cylinder, and a discharge mechanism for driving the ram through the penetrable wall section of the gas cylinder when a trigger device is released. Discharge of the pressurized gas from the cylinder drives the plunger to effect a jet injection, and also drives the seal of the gas cylinder to effect resetting of the discharge mechanism. The shell with its plunger, and spent gas cylinder, is discarded after an injection; and a new shell pre-filled with medication and with a new gas cylinder is used for each injection.

The Parsons '699 patent is believed to teach a single-use jet injector which is totally discarded after one use. This injector is believed to have a body with a pair of gas chambers separated by a breakable valve. One of the gas chambers contains a pressurized gas, while the other chamber is sealingly bounded by a piston which drives a plunger. The plunger sealingly bounds a chamber into which a dose of medication is loaded by the user before the injection. This medication dose chamber leads to an injection orifice so that when the valve is broken, the piston and plunger are moved by pressurized gas communicated to the second chamber, and the plunger drives the medication forcefully out of the injection orifice to form an injection jet. After a single use, the device is discarded.

The Castellano '723 patent, which was issued in 1998 and which does not cite the earlier Parsons '699 patent, is believed to teach substantially the same subject matter as Parsons et al.

WIPO publication WO 97/37705 published pursuant to a Patent Cooperation Treaty (PCT) application for joint inventors Terence Weston and Pixey Thornlea, is believed to disclose a disposable hypodermic jet injector in which the device is powered by a gas pressure spring of the type common in the tool and die art as a substitute for the conventional metal spring-powered ejector pin. In the Weston device, the ram of the gas pressure spring is held in a contracted position by a trigger mechanism. When the trigger mechanism is released, the gas pressure spring is supposed to expand and drive a piston sealingly received in a bore and leading to a fine-dimension orifice in order to produce a jet hypodermic injection from liquid held in the bore ahead of the piston.

The Weston device is thought to have several deficiencies: such as difficult and costly manufacturing and sterilization processes, because pressurized gas and a drug dose need to be contained in the same package; and including a possible inability to endure long-term storage while still retaining the gas pressure in the gas spring to power an injection, and also maintaining the medication integrity. In other words, the gas pressure spring of the Weston device contains only a small quantity of gas, and depends upon the sealing relationship of the ram of this spring with a cylinder within which the ram is movably and sealingly received in order to retain this gas pressure. Even a small amount of gas leakage over time will be enough to render this injector inoperative.

### Summary of the Invention

In view of the above, it is desirable and is an object for this invention to provide a needle-less hypodermic jet injection device which reduces the severity of or avoids one or more of the limitations of the conventional technology.

Thus, it is an object of this invention to provide a single-use, disposable, needle-free gas-powered hypodermic jet injector utilizing a pressurized gas source which is hermetically sealed until the moment of injection.

Further, an object of this invention is to provide such a gas powered jet injector in which the device has a storage configuration and a use configuration. In the storage configuration, the device is safe, with the drug cartridge sealed closed, and is incapable of effecting a jet injection. In the use configuration, the device is prepared for making a jet injection, with the drug cartridge opened in preparation for this injection.

Additionally, an object for this invention is to provide such an injection device having a multi-function component which alternatively maintains the injector in a safe storage condition, and also allows a user to place the injection device into a use condition preparatory for performing a jet injection. When the user placed the device into the use configuration, the multi-function component prepares the jet injection device by effecting unsealing of the previously sealed drug cartridge, and also removes a safety block from an obstructing position relative to a trigger of the device. Thereafter, the trigger of the injector can be manually activated by a user of the device to perform an injection.

According to the present invention there is provided a needle-less hypodermic jet injection device according to claim 1 or claim 2.

Accordingly, a needle-less hypodermic jet injection device embodying this invention includes, for example: a hand piece assembly having a body including a drug injection cartridge with a medication cylinder pre-tilled with substantially incompressible liquid medication such that substantially no ullage volume exists in the medication cylinder, the medication cylinder leading to an outlet orifice a plug-capture chamber and a drug injection nozzle, a sealing member sealingly and movably received in the outlet orifice, and a drug-injection piston; the hand piece assembly further defining a first bore within the body for movably receiving a gas-power piston, a gas power piston movably received in the first bore and having a ram portion extending into the drug injection cartridge to abut with the drug-injection piston, the body and gas-power piston cooperating to define a first variable-volume chamber in the first bore; the body also defining an elongate second bore in gas communication with the first bore and separated therefrom by a center wall portion of the body, a cylindrical gas capsule received into the second bore, the gas capsule having a penetrable wall section disposed toward the center wall, the center wall carrying a penetrator disposed toward the penetrable wall section of the gas capsule, and the hand piece assembly carrying a discharge mechanism including a trigger member outwardly disposed on the body and a hammer movable in the body in response to actuation of the trigger to forcefully move the gas capsule in the second bore so as to impale the gas capsule at the penetrable wall section thereof upon the penetrator and thus to communicate pressurized gas to the first chamber; whereby, the pressurized gas in the first chamber drives the gas-power piston to effect a hypodermic jet injection from the drug injection cartridge, and the body and trigger member cooperatively defining a first relative position in which the ram portion confronts but does not displace the injection piston so that the sealing member is disposed in the outlet orifice to maintain the drug injection cartridge sealingly closed, and the body and trigger member in a second relative position preparatory to effecting a jet injection causing the ram portion to abut and move the drug injection piston to a second position displacing the drug injection piston to a second position so that the sealing member is displaced from the outlet orifice into the plug-capture chamber by the liquid medication and unseals the drug injection cartridge.

Preferred additional features of the invention are defined in claims 3 to 20 preferred embodiments of the invention include the following. A needle-less hypodermic jet injection device comprising a pre-filled drug injection cartridge including a medication cylinder having an outlet orifice, an injection nozzle, a flow path communicating the outlet orifice to the injection nozzle, a plug member in a first position sealingly disposed in the flow path, a drug-injection piston in a first position cooperating with the medication cylinder to define a variable-volume chamber of first selected size, and a dose of substantially incompressible liquid medication substantially filling the variable-volume chamber at the first size with substantially no ullage volume. The drug-injection piston having a second position cooperating with the medication cylinder to define a variable-volume chamber of second selected size smaller than the first selected size, so that the incompressible liquid medication displaces the plug member from the first position of sealing disposition in the flow path to a second position of capture in the flow path, the medication cylinder and the plug member in the second position thereof cooperatively defining an open flow path between the variable-volume chamber and the injection nozzle. A hand piece assembly having a body holding the drug injection cartridge, the hand piece assembly including means for forcefully moving the drug injection piston from the second position to a third position so as to reduce the volume of the variable-volume chamber substantially ejecting the dose of liquid medication via the injection nozzle. The hand piece assembly further including a first body portion holding the drug injection cartridge, and an abutment member selectively movable into engagement with the drug injection piston to move the drug injection piston from the first position to the second position.

Additional objects and advantages of this invention will appear from a reading of the following detailed description of a single exemplary preferred embodiment, taken in conjunction with the appended drawing Figures, in which the same reference numeral is used throughout the several views to indicate the same feature, or features which are analogous in structure or function.

### Brief Description of the Drawing Figures

Figure 1 provides an exterior side elevation view of a single-use, needle-less hypodermic jet injector device embodying the present invention, and in which the device is in a "storage" configuration;
Figure 2 is an exterior side elevation view of the injector device seen in Figure 1, but with the device shown in an "inject" configuration preparatory to effecting a hypodermic jet injection;
Figure 3 provides a longitudinal cross sectional view through the needle-less hypodermic jet injection device of Figure 1, and shows the device in the "storage" configuration;
Figure 4 is a fragmentary cross sectional view similar to Figure 3, but shows the hypodermic jet injection device in the "inject" configuration;
Figure 5 is also a fragmentary cross sectional similar to Figures 3 and 4, but shows the hypodermic jet injection device during the process of effecting a jet injection;
Figure 6 is a fragmentary cross sectional view similar to a portion of Figure 4, but shows a respective portion of an alternative embodiment of a single-use, needle-less hypodermic jet injection device according to the present invention;
Figure 7 is a perspective view of a portion of the device seen in Figure 6;
Figure 8 provides a cross sectional view of the portion of the device seen in Figure 7;
Figure 9 provides an exterior side perspective view of an alternative embodiment of a single-use, needle-less hypodermic jet injector device embodying the present invention, and in which the device is in a "storage" configuration;
Figure 10 provides a longitudinal cross sectional view through the needle-less hypodermic jet injection device of Figure 9, and shows the device in the "storage" configuration;
Figure 10a is a fragmentary cross sectional view taken along line a-a of figure 10;
Figure 10b is a fragmentary longitudinal cross sectional view of a portion of the device of Figure 10, with portions removed or broken away for clarity of illustration, with cooperating parts shown in their "storage" positions, and is shown at an enlarged size;
Figure 11 is an exterior side perspective view of the injector device seen in Figure 9, but with the device shown in an "inject" configuration preparatory to effecting a hypodermic jet injection;
Figure 12 is a longitudinal cross sectional view similar to Figure 10, but shows the hypodermic jet injection device in the "inject" configuration;
Figure 12a is a fragmentary longitudinal cross sectional view similar to Figure 10b, but with cooperating parts in their "inject" positions, and is also shown at the same enlarged size;
Figure 13 provides an exploded perspective view of parts of the injection device of Figures 9-12;
Figure 14 is a greatly enlarged fragmentary cross sectional view similar to a portion of Figure 12, but shows the hypodermic jet injection device during the process of effecting a jet injection;
Figure 14a is a fragmentary cross sectional view taken at plane 14a-14a of Figure 14; and
Figure 15 is a longitudinal cross sectional view similar to Figures 10 and 12, but showing the device after completion of an hypodermic jet injection.

### Detailed Description of Exemplary

### Preferred Embodiments of the Invention

### Overview, storage of the device, and its

### Preparation for effecting a jet injection

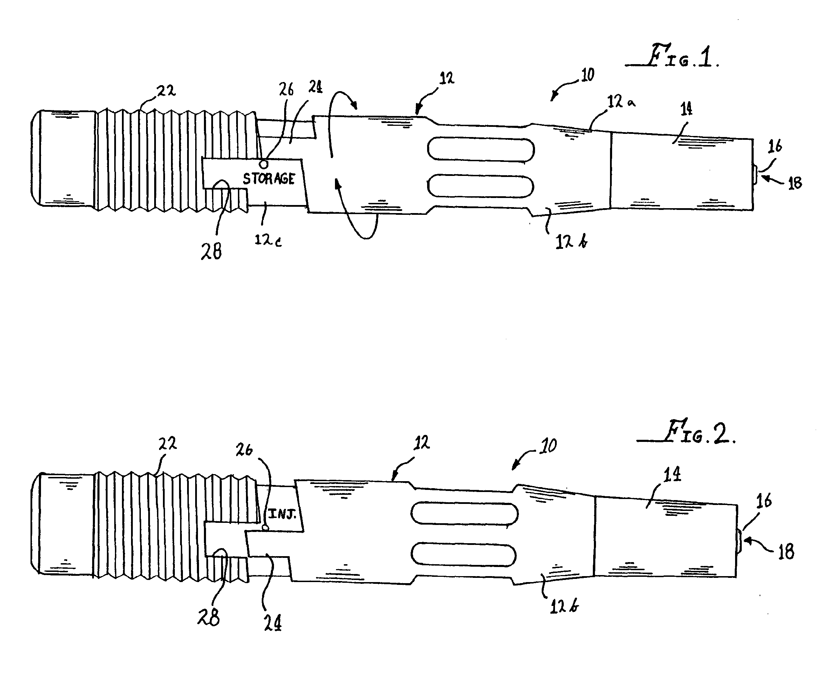

Viewing Figure 1, a needle-free, hypodermic jet injection device 10 is shown in a storage configuration in which it is maintained until it is prepared for its use in administering an injection. In this storage configuration, the device is incapable of effecting a jet injection, is safe, and can be stored for a comparatively long time while requiring only a moment of preparation before it can be used to make a jet injection of the medication within the device 10.

The device 10 includes a hand piece assembly 12, preferably fabricated principally of injection molded plastic polymers, and with a body 12a including a pre-filled drug injection cartridge 14. The word "drug" as used herein is intended to encompass, for example, and without limitation, any medication, pharmaceutical, therapeutic, vaccine, or other material which can be administered by jet injection. Essentially, such an injectable medication is in the form of a substantially incompressible liquid, and as will be seen, this liquid substantially fills the drug injection cartridge so that no ullage volume of compressible gas is present in this cartridge.

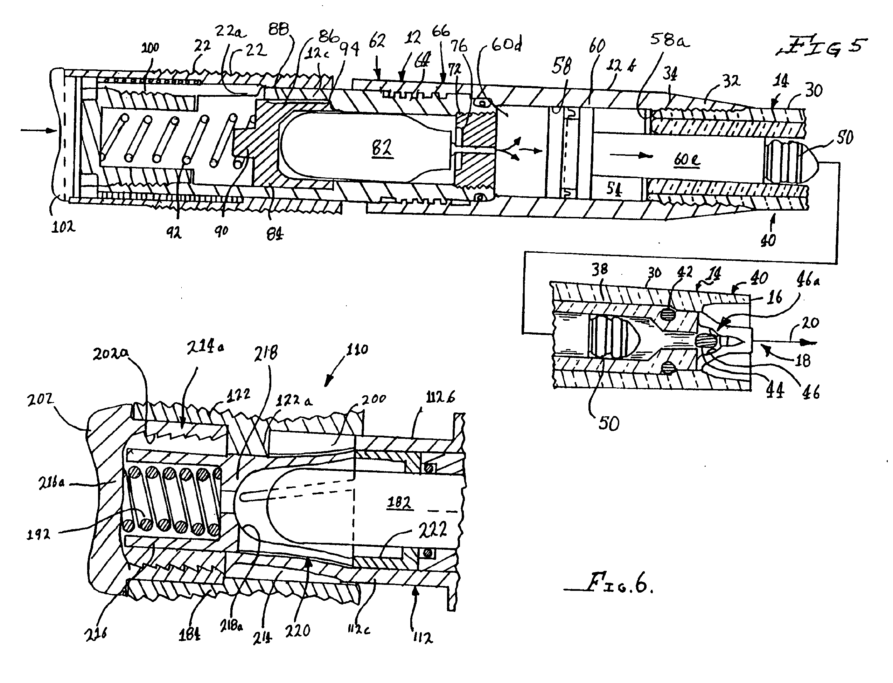

The pre-filled drug injection cartridge 14 has an end surface 16 at which is defined a fine-dimension injection orifice opening 18. When the device 10 is used to effect an injection, a high velocity jet of liquid medication issues from this orifice (as is indicated by arrow 20 of Figure 5). To use the device 10, it is first placed in an "inject" configuration, the end surface 16 is pressed against the skin of a patient who is to receive the jet injection, and then the device 10 is triggered so that the jet 20 issues out and penetrates the skin. Thus, the liquid medication enters the tissues of the patient without the use of a hypodermic needle.

Placing the device 10 in the "inject" configuration is effected manually by a user of the device 10 who rotates a first portion 12b of the body 12a relative to a second portion 12c. As is seen in Figure 1, the body portion 12c carries a trigger sleeve 22, while the portion 12b carries a projection 24 abutting this sleeve. The projection 24 and a blocking pin 26 cooperate to prevent the body portions 12b and 12c from being relatively rotated except in the direction of the arrow of Figure 1. When a user effects this relative rotation of the body portions 12b and 12c through a rotation of almost 360°, then this relative rotation aligns the projection 24 with a recess 28 on the trigger sleeve 22, reveals the abbreviation of the word "inject" (indicated on Figure 2 by the letters "INJ") on the body portion 12c.

This relative rotation of the body portions 12b and 12c also effects a selected relative axial movement of these body portions toward one another (as will be further described below), and places the device 10 in the "inject" configuration seen in Figure 2. In this "inject" configuration, the device 10 is positioned with its surface 16 against the skin of the person who is to receive the injection, and an axial pressure is applied to the trigger sleeve 22. The trigger sleeve 22 moves axially along the body portion 12c, and this movement triggers the device 10 to effect injection jet 20 (recalling Figure 5).

### Structure of the device 10

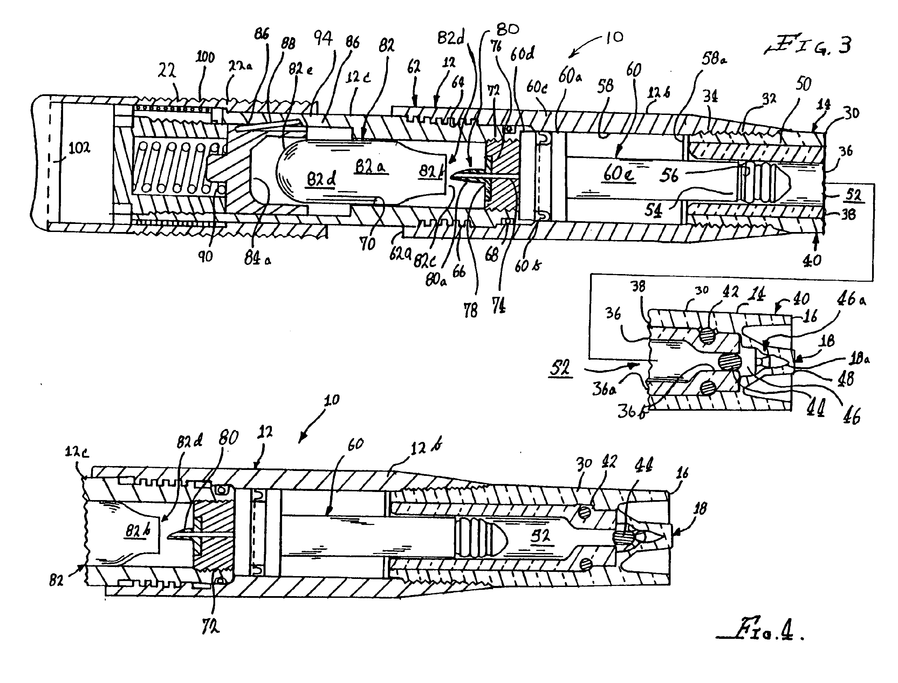

Turning now to Figures 3, 4, and 5, in conjunction with one another, Figure 3 shows the device 10 in the storage configuration of Figure 1 preparatory to giving an injection. In Figure 4 shows the device in the "inject" configuration, and Figure 5 shows the device during the brief interval of an injection. In these Figures, it is seen that the drug cartridge 14 includes a cylindrical body 30 defining an external thread section 32. This external thread 32 is threadably received by a matching internal thread section 34 of the body portion 12b. Preferably, a thread locking compound, such as an anaerobic adhesive, is applied to the threads 32 of the cartridge 14 when it is assembled to the body portion 12b during manufacture of the device 10. Alternatively, a self-locking thread design or a thread-locking feature may be used on the device 10 to prevent the drug injection cartridge 14 from being removed from the device 10. Thus, the cartridge is not removable from the device 10, and the device 10 and cartridge 14 are disposed of after the first and only injection effected with the device 10.

An advantageous feature of the device 10 embodying the present invention, and one which results from this construction of the device, is that the injection cartridge 14 may be manufactured and filled at a drug company (without the drug manufacture having to be concerned with handling capsules of pressurized gas), the gas pressure capsule of the device may be manufactured and filled at a factory devoted to this item (without this manufacturer having to handle drugs), and the hand piece assembly of the device may be manufactured at yet another location, if desired. Subsequently, completion of the device 10 requires merely the combining of the hand piece assembly, gas capsule, and drug injection cartridge.

The body 30 of cartridge 14 defines a stepped through bore 36 having a larger diameter portion 36a which extends substantially the length of the body 26. Adjacent to the forward end of the body 30 (i.e., adjacent to the end defining surface 16), the bore 36 steps down and defines an outlet orifice 36b. It is seen that the bore portion 36a and outlet orifice 36b are defined by a glass sleeve 38 which is received into a molded plastic body 40. An O-ring type of seal member 42 prevents leakage between the glass sleeve 38 and the body 40.

As those who are ordinarily skilled in the pertinent arts will understand, many medications are not suitable for long-term storage in contact with plastics, but will store satisfactorily in contact with glass. Thus, this construction of the cartridge 14 makes it suitable for long-term storage of even medications of this nature. However, for medications that will store satisfactorily in contact with plastic polymers, this construction detail is optional and the entire injection cartridge body 30 may be formed of a selected polymer.

In the embodiment of cartridge 14 having the glass sleeve 38, the outlet orifice 36b is sealingly closed in the storage configuration of the device 10 by a plug 44. Importantly, viewing Figures 3-5, it is seen that the cartridge 14 defines a plug-capture chamber 46 immediately outside of the outlet orifice 36b (i.e., rightwardly of this outlet orifice, viewing Figures 3-5). The plug capture chamber 46 includes a radial array 46a of individual radially inwardly and axially extending ribs 48 disposed in a spaced relation to the outlet orifice 36b. These ribs 48 are arrayed radially about and in a transition bore portion 18a leading to the injection orifice 18. Thus, as will be seen, the plug member 44 can be received into the plug-capture chamber 46 and be supported on the ribs 48 without it blocking the injection orifice 18.

Sealingly and movably received in the bore section 36a is a resilient piston member 50. This piston member defines multiple circumferential grooves 50a interdigitated with sealing ribs 50b. The sealing ribs 50b sealingly and movingly engages the bore 36a of the injection cartridge (i.e., with the bore 36a of glass sleeve 38 in this case). The piston member 34 and body 30 cooperatively define a medication chamber 52 communicating outwardly of the cartridge 14 via the injection orifice 18. Prior to its use to effect an injection, the orifice 18 of each fresh and pre-filled device 10 will ordinarily also be sealed by an adhesively-applied, peel-off type of sealing membrane, which may be formed, for example, of foil or of a polymer/paper laminate. Such peel-off seals are conventional and well known, and for this reason, the seal formerly on cartridge 14 of device 10 as seen in Figure 3 is not shown in the drawing Figures.

Further considering the cartridge 14, it is seen that the piston member 50 defines an abutment surface 54 confronting the opening of bore 36 on body 30. This surface 54 is abutted by an end surface 56 on an injection ram of the hand piece assembly 12 (which injection ram will be further described below). In the storage configuration of the device 10, the end surface 56 confronts piston 50, but does not displace it from the position seen in Figure 3. In this storage configuration of the device 10, the chamber 52 is sealed and is substantially full of incompressible liquid, without any substantial ullage volume of compressible gas being in the chamber 52. The injection ram will be understood as effective during a jet injection to forcefully move the piston 50 inwardly of the bore section 36a toward the outlet orifice 36b

### Hand piece assembly 12

Considering now the hand piece assembly 12 in greater detail, as seen in Figures 1-5, it is seen that the body 12a generally is formed of two main cooperative tubular sections 12b and 12c, which are threadably engaged with one another to form the hand piece assembly 12. Preferably both of the body sections 12b and 12c, as well as other components of the device 12 not otherwise identified as being made of some other material, are all formed of plastic polymers. Further, the preferred process for making the device 10 is by injection molding of the components formed of plastic polymer, so that manufacturing costs are very low. Materials utilization for the device 10 is very small as well, so that disposing of the device after a single injection does not cause a serious environmental concern.

The forward tubular body section 12b defines a stepped through bore 58, a forward portion 58a of which opens at 58b forwardly on the body 12, and which inwardly of this bore opening 58a defines the internal thread section 34 for threadably receiving the external threads 32 on the drug cartridge 14. Sealingly and movably received in the bore portion 58a is a stepped injection piston member 60. A larger diameter portion 60a of this piston member defines a groove 60b carrying a seal member 60c. The seal member 60c movingly engages sealingly with the bore portion 58a and bounds a gas pressure chamber 60d, which is to the left of this piston member as seen in Figures 3, 4, and 5. It is to be noted that in Figures 3 and 4, this chamber 60d is at a minimal volume, and so the lead line from reference numeral 60d extends into the interface of the piston member 60 with the housing portion 12c.

A smaller diameter portion 60e of the piston member 60 is elongate and extends in the bore 58 to also be received into the bore portion 36a of the drug cartridge 14, as is seen in Figure 3 in the storage configuration of the device 10. The piston portion 60e defines the end surface 56 which confronts and abuts the surface 54 of the piston member 50 of an drug cartridge 14. Thus, the piston portion 60e provides the injection ram of the device 10.

Considering the forward body section 12b in still greater detail, it is seen that this body section defines a tubular aft body section 62. This aft body section includes an axially disposed end surface 62a at which the stepped through bore 58 opens, and which defines an internal thread section 64 threadably engaging onto matching threads 66 of body section 12c. For purposes of explanation, and without limitation of the present invention, the threads 64 and 66 may have a pitch of about 14 threads per inch.

As is seen comparing Figures 1 and 2, the device 10 is converted from its storage to its "inject" configuration by rotating the body portions 12b and 12c in a relative rotational direction that threads these body portions together along threads 64 and 66. As was explained above, this relative rotation of the body sections 12b and 12c brings projection 24 into alignment with recess 28 on trigger sleeve 22, and makes possible the subsequent triggering of the device 10.
Still considering Figures 2 and 3, it is seen that the aft body portion 12c outwardly defines the thread section 66 and slidably carries the trigger sleeve 22. Adjacent to the thread section 66, the body portion 12c carries an O-ring type of sealing member 68 which sealingly engages the body portion 12b both when the body portions are in their "storage" relative configuration of Figure 3, and also when these body portions are in their "inject" relative positions as is seen in Figures 4 and 5.

Body portion 12c defines a stepped through bore 70 which is substantially closed at the end of this bore adjacent to the forward body portion 12b by a wall member 72. This wall member 72 defines a stepped through bore 74 in a larger diameter part of which is seated a disk part 76 of a penetrator member 78. This penetrator member 78 includes a hollow penetrator spike 80 which itself has a bore 80a communicating through the wall member 72 via the smaller diameter portion of bore 74. Thus, the bore 70 is communicated to the chamber 60d adjacent to injection piston 60 in the body portion 12b.

Slidably received in the bore 74 adjacent to and confronting the penetrator member 78 is a gas pressure capsule 82. This gas pressure capsule 82 includes a body 82a, having a cylindrical outer wall portion 82a'. The capsule 82 is also necked down at a forward end to provide a reduced diameter portion 82b leading to an axially disposed end surface 82c defined by a penetrable wall section 82d (the wall section being indicated by the arrowed numeral in Figure 3). The gas capsule 82 is preferably formed of metal, and contains a supply of pressurized gas. Because the pressurized gas is contained in the capsule 82 until the moment of injection, the plastic parts of the device 10 are not exposed to or stressed by this pressurized gas until an injection is effected using the device 10. For this reason, the device 10 is believed to have a much more reliable storage life then prior devices which attempt to contain pressurized gas in a plastic or plastic-composite containment.

The wall section 82d confronts and is spaced slightly from the penetrator spike 80. At an opposite or aft end of the capsule 82, this capsule defines an outwardly rounded end wall 82e.

Also slidably received into the bore 70 and confronting the end 82e of capsule 82 is tubular and cylindrical hammer member 84. This hammer member 84 defines an end surface 84a which is engageable with the surface 82e of capsule 82, an axially extending groove 86 having an end wall at 86a (into which a dowel pin 88 is received), and an axial protrusion at 90 which serves to center a spring 92.

The dowel pin 88 is engaged in a first position (i.e., in the "storage" configuration of the device 10) at end 86a of groove 86, and the other end of this pin rests upon a metal (i.e., preferably hardened steel) sear pin 94 carried by the body portion 12c. Thus, as is seen in Figures 3 and 4, the hammer 84 is maintained in a "cocked" position with the spring 92 pre-loaded between the hammer 84 and a spring seat member 96 threadably engaging into the end of body portion 12c.

In order to provide for movement of the trigger sleeve 22 to effect release of the hammer 84, the body portion 12c defines an axially extending slot 100, and the trigger sleeve 22 carries a radially inwardly extending trigger block 22a, which is slidably received in this slot 100 and which confronts the dowel pin 88, as is seen in Figure 3. Also, an end cap 102 is adhesively retained onto the trigger sleeve 22 and closes the end of this trigger sleeve so that a user's thumb, for example, may be used to effect forward movement of the trigger sleeve when an injection is to be effected. It will be understood that the trigger sleeve 22 may alternatively be grasped between the thumb and fingers, for example, to position the device 10 for making an injection, and then effecting forward movement of the trigger sleeve 22 to effect this injection.

However, as was pointed out above in connection to the comparison of Figures 1 and 2, the device 10 is first placed by a user into its "inject" configuration before a jet injection can be effected. This conversion of the device 10 from its "storage" configuration to its inject configuration is effected by relative rotation of the body portions 12b and 12c, as is indicated by the arrow on Figure 1. As is seen in Figure 2, this relative rotation of the body portions 12b and 12c brings the projection 24 into engagement with blocking pin 26 and into alignment with recess 28, so that the trigger sleeve 22 is movable in the axial direction toward body portion 12b. However, viewing Figure 4, it is seen that this relative rotation of the body portions 12b and 12c also threads body portion 12c by substantially one thread pitch dimension into the body portion 12b.

Because the body portion 12c and wall member 72 are abutting injection piston member 50, this piston member 50 is moved rightwardly, viewing Figure 4, by substantially one thread pitch dimension. Consequently, the ram portion 60e of the injection piston 60 moves forward and forces piston 50 forwardly by a sufficient amount that plug member 44 is dislodged hydraulically (recalling that the liquid medication in chamber 52 is substantially incompressible) from the outlet orifice 36b and into plug-capture chamber 46. In this chamber 46, the plug member 44 is retained an rests upon the ribs 48 while these rib provide a flow path leading around the plug member 44 from the outlet orifice 36b to the injection orifice 18.

Although the conversion of device 10 from its "storage" configuration to its "inject" configuration unseals the injection cartridge 14, this is not detrimental to the integrity of the medication in chamber 52 because it happens mere moments before the device 10 is used to inject the medication into a patient. This injection is effected by placement of the device 10 with its surface 16 against the skin at the intended location of injection, and sliding of trigger sleeve 22 forward (which also assists in seeing that the device 10 is held firmly to the skin), so that the trigger block 102 slides along slot 100 to dislodge the dowel pin 88 from sear pin 94, viewing Figure 5.

As is seen in Figure 5, the result is that the hammer member 84 is driven forward by spring 92, impacts the capsule 82, and impales this capsule at penetrable wall 82d, as is seen in Figure 5. The result is the penetrator spike 80 penetrates the wall 82c of the capsule 82, and allows pressurized gas from this capsule to flow along the bores 80a and 74 into the chamber 60d. This pressurized gas in chamber 60d drives piston member 60 forwardly, so that the piston 50 in bore 36a is also driven forwardly. Forward movement of piston 50 drives the liquid medication out of chamber 52, past the plug member 44 in plug-capture chamber 46, and out of injection orifice 18, forming injection jet 20.

After the jet injection depicted in Figure 5, the device 10 is disposed of by the user of the device, and it is not again used. That is, the device 10 is a single-use device and is not designed or intended to be recharged or refilled. This design of the device 10 insures safety for those receiving an injection by use of the device 10 because they can be sure that only a new and never before used device is used to give them the injection. Further, the device 10 provides for long-term storage of the device and its pre-filled medication, so that devices 10 may be stockpiled in anticipation of such events as mass inoculations. The device 10 may be used under exigent circumstances as well, since it requires only a few seconds or less to convert it from its "storage" configuration to its "inject" configuration, after which the jet injection is immediately effected.

Figure 6 provides a fragmentary view of an alternative embodiment of the jet injection device according to this invention. In Figure 6, only the aft or trigger assembly end of the device is illustrated. The forward end of the device and its pre-filled medication injection cartridge may be substantially as depicted and described above. Because the device illustrated in Figures 6-8 has many features that are the same as, or which are analogous in structure or function to those illustrated and described above, these features are indicated on Figures 6-8 using the same reference numeral used above, and increased by one-hundred (100).

Viewing Figures 6-8 in conjunction with one another, it is seen that the injection device 110 includes a body portion 112c, which is necked to a slightly smaller diameter aft portion at 214. This aft portion defines a plurality of circumferential barbs 214a, and an end cap 202 is received on these barbs and is permanently engaged there by a matching set of inwardly extending barbs 202a. Slidably received in this body portion 112c is a one-piece molded hammer-and-sear member 184.

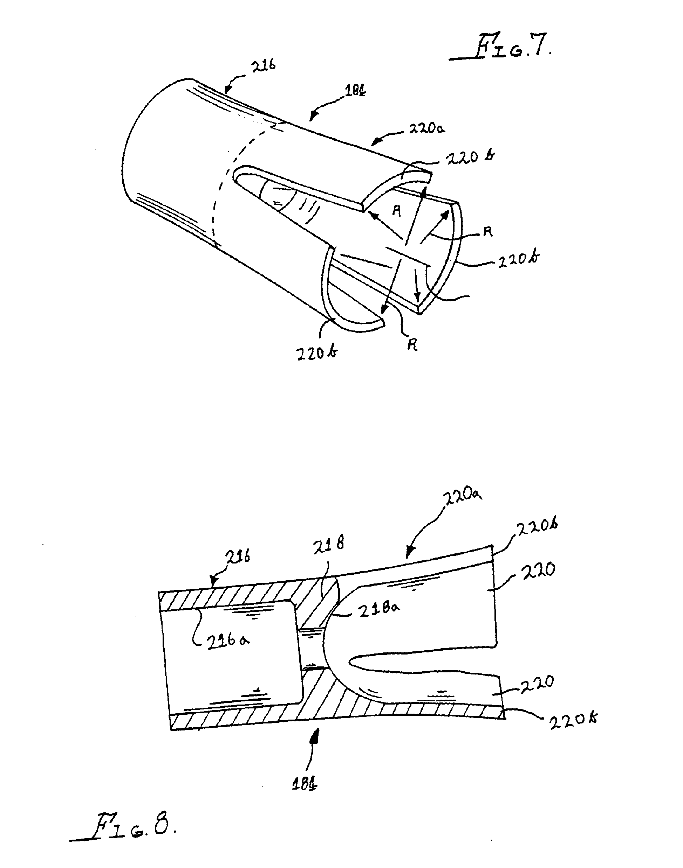

Preferably, this member 184 is molded of plastic polymer. The hammer-and-sear member 184 is seen in perspective in Figures 7 and 8. It is seen that this hammer-and-sear member 184 includes a cylindrical section 216 defining a spring recess 216a, into which the spring 192 is captively received and preloaded to make the device 110 ready for use. A center wall portion 218 of the member 184 provides a surface 218a, which is engageable with the gas capsule 182 to move this capsule forward, and to impale the capsule on the penetrator spike (not seen in figure 6, but recalling Figures 3-5 above). In order to hold the hammer-and-sear member against the pre-load of spring 192, and to resist the pressure of this spring over a long term the member 184 includes three axially extending legs 220.

Each of these legs 220 is a portion of a cone-shaped section 220a, best seen in Figures 7 and 8. The transition between the circular cylindrical section 216, and the cone-shaped section 220a is indicated with a dashed line circumscribing the member 184 in Figure 7. Forwardly of this transition, the legs 220 flare out by their own resilience. As is seen in Figure 6, these legs 220, at an end surface 220b of each one engage upon a ring-like abutment member 222 carried within the body portion 112c. As is best appreciated by consideration of Figure 7, it is seen that the end surfaces 220b of the legs 220 are not formed on the radius of the cone-shape at this end of the member 184 (i.e., at the cone diameter having a center line indicated as "CL" on Figure 7), but are formed at a smaller radius corresponding generally with the circular diameter of the section 216 (indicated by the radius lines and character "R" of Figure 7). During storage of the device 110, these end surfaces 220b rest upon the abutment member 222 and transfer the spring force from spring 192 to this abutment member on a long-term basis.

In order to prevent creep of the plastic polymer material from which the member 184 is formed, the surfaces 220b define cooperatively, a contact area which corresponds substantially to that of the diameter 216 of the member 184 multiplied by the radial thickness of the legs 220. This contact surface area is sufficient to prevent creeping of the polymer from which the member 184 is formed.

In order to effect release of the hammer-and-sear member 184 when it is desired to effect a jet injection with the device 110, the body portion 112c defines three axially extending slots 200 (only one of which is seen in Figure 6), each corresponding to a respective one of the legs 220. As is seen in Figure 6, the trigger sleeve 122 carries three trigger blocks 122a (again, only one of which is seen in Figure 6) which are slidably received in the slots 200. When this trigger sleeve 122 is moved forward, the trigger blocks 122a simultaneously force respective ones of the legs 220 radially inwardly and out of engagement with the abutment member 222, overcoming both the inherent resilience of these legs and the component of spring force resulting from the radial flaring of these legs. It will be appreciated that in view of this combination of inherent resilience and outward flare of the legs 220, there is virtually no risk that the device 110 will trigger except in response to deliberate forward movement of the trigger sleeve 122.

Because the legs 220 are formed at a circular (rather than conical) radius, they nest together and are received into the ring-like abutment member 222. Thus, the spring 192 forces the hammer-and-sear member 184 forcefully forward, effecting a jet injection from the device 110, as was explained above.

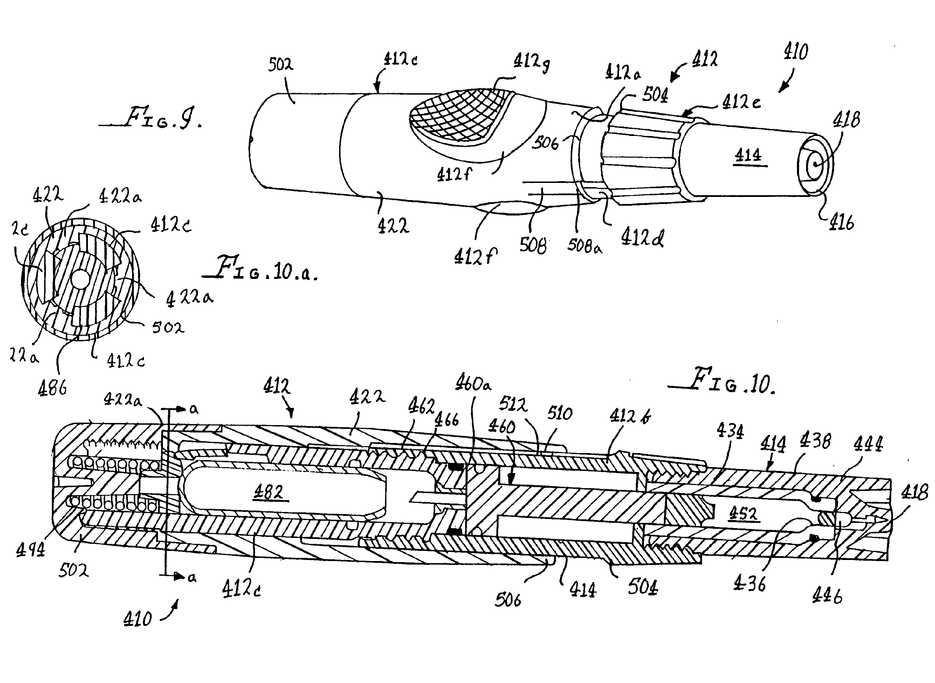

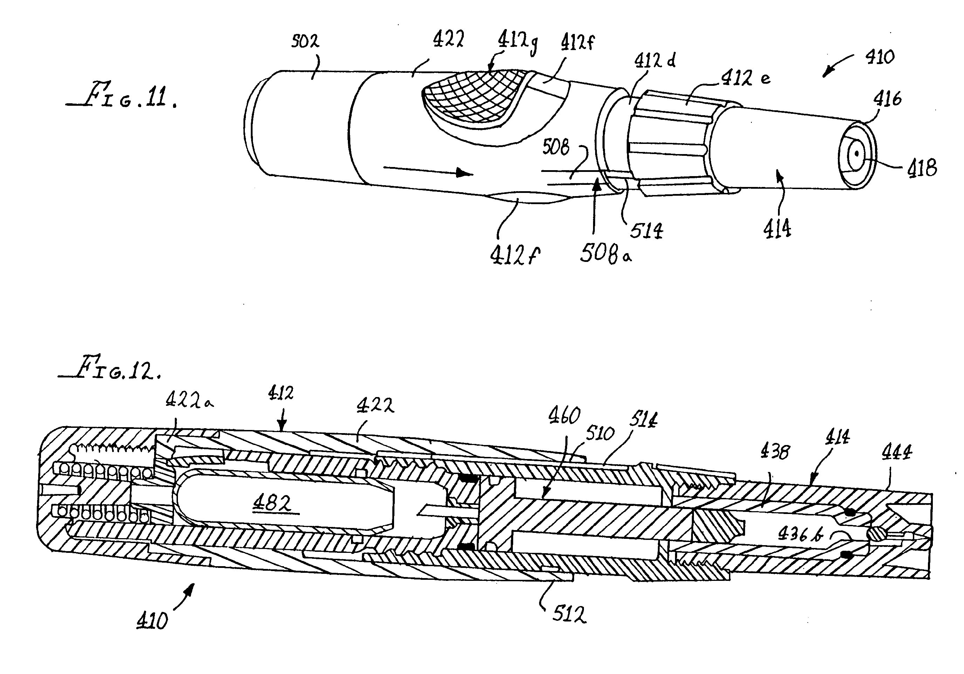

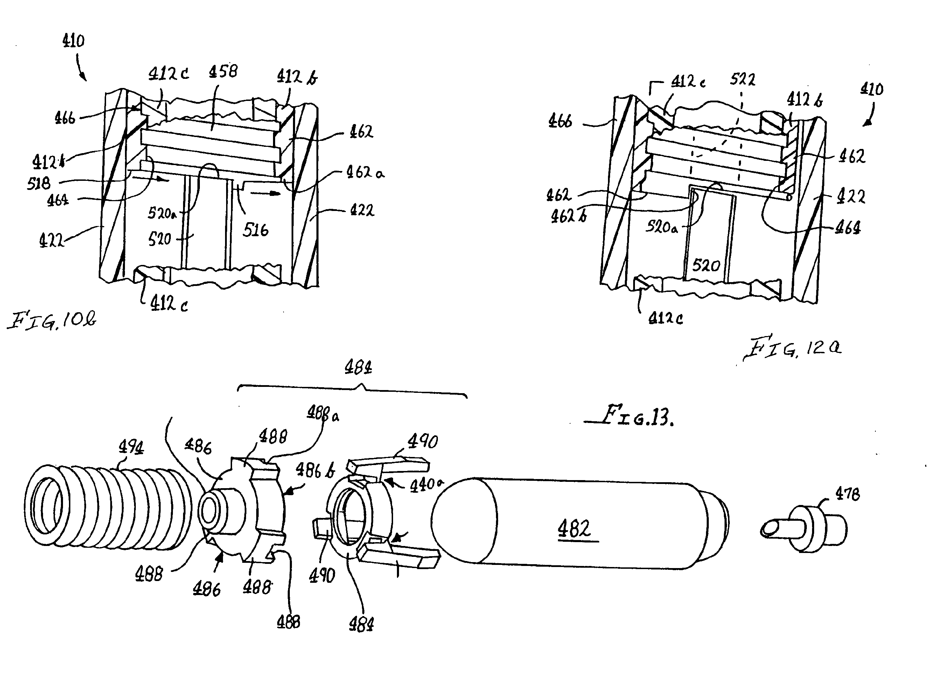

Viewing now Figures 9-15, yet another alternative embodiment of a needle-free, hypodermic jet injection device is shown. Because the device illustrated in Figures 9-15 has many features that are the same as, or which are analogous in structure or function to those illustrated and described above, these features are indicated on Figures 9-15 using the same reference numeral used above, and increased by four-hundred (400). In Figures 9, 10, 10a, and 10b, the device 410 is shown in a storage condition. On the other hand, figures 11, 12, and 12a show the device 410 in an "inject" condition preparatory to the effecting of a hypodermic jet injection using the device.

Viewing first Figures 9, 10, 10a and 10b, it is seen that the device 410 is in a storage configuration in which it is maintained until it is prepared for its use in administering an injection. The device 410 includes a hand piece assembly 412, preferably fabricated principally of injection molded plastic polymers, and a including a pre-filled drug injection cartridge 414 with an end surface 416 at which is defined an injection orifice 418. The cartridge 414 has a glass sleeve 438, and an outlet orifice 43 6b, which is sealingly closed in the storage configuration of the device 410 by a plug 444.

In this embodiment, the plug 444 preferably takes the form of a ball member, forcibly and sealingly received into the outlet orifice 436b. This ball member 444 is more preferably formed of Teflon material (i.e., Polytetrafluoroethylene) in order to sealingly close the outlet orifice 436b, to provide a chemically inert plug member for the cartridge 414, and to facilitate a dependable and repeatable level of force required to dislodge this plug member from the orifice 436b. Importantly, viewing Figure 10, it is seen that the cartridge 414 also defines a plug-capture chamber 446 immediately outside of the outlet orifice 436b from the glass sleeve 438. Further to the discussion above, it will be appreciated that the plug capture chamber 446 provides a "bypass" passage for outward flow of the medication in the cartridge 414 around the plug member ball 444 when this ball is in this chamber. In this embodiment, the chamber 446 takes the form of a concave or spherical cavity having also a plurality of bypass passages 448 (only one of which is seen in the drawing Figures) allowing bypass flow of the liquid medication past the plug member 444 when this plug member is in the chamber 446.

Considering now the hand piece assembly 412 in greater detail, as shown in Figures 9-10b, it is seen that the body 412a generally is formed of two main cooperative tubular sections 412b and 412c, which are threadably engaged with one another to form the hand piece assembly 412. The forward tubular body section 412b defines a stepped through bore 458, a forward portion 458a of which opens at 458b forwardly on the body 412, and which inwardly of this bore opening 458a defines the internal thread section 434 for threadably receiving the external threads on a drug cartridge (recalling the description above).

Sealingly and movably received in the bore portion 458a is a stepped injection piston member 460. A larger diameter portion 460a of this piston member defines a groove 460b carrying a seal member 460c sealingly engaging with the bore portion 458a to bound a gas pressure chamber 460d.

Again, considering the forward body section 412b in detail, it is seen that this body section defines a tubular aft body section 462 which has an axially disposed end surface 462a at which the stepped through bore 458 opens. Inwardly of this opening, the body section 462 defines an internal thread section 464 threadably engaging onto matching threads 466 of body section 412c. The body portion 412c carries a trigger sleeve 422, but the body portion 412b does not outwardly carry features like the projection 24, blocking pin 26, or recess 28 described above.

The present embodiment of injector 410 defines a pair of spaced apart axially disposed confronting annular surfaces 504, 506, one of which is defined on the forward body portion 412b, and the other of which is defined on the trigger sleeve 422. The surface 506 is interrupted by an axially extending resilient detent finger 508, the forward distal end portion 508a of which defines a radially inwardly extending nub 510. This radially inwardly extending nub 510 is removably received into a recess 512 defined in the radially outer surface of forward body portion 412c. The nub 510 and recess 512 have at least one sloping surface so that the body portions 412b and 412c can be relatively rotated manually from the position seen in Figure 9 to that of Figure 11 in the direction of the arrows of Figure 9. Because of the at least one sloping surface on the recess 512 and/or nub 510, in response to this relative rotation of the body portions 412b and 412c (recalling that body portion 412c rotates in unison with trigger sleeve 422), it is seen that the detent finger 508 is forced from the recess 512 and rides about a cylindrical outer surface portion 412d of body portion 412b as the portions 412b and 412c are relatively rotated. As Figure 11 illustrates, upon the two body portions 412b and 412c having been relatively rotated through about 180°, the nub 510 falls (actually "snaps") into an axially extending guide groove 514 defined by body portion 412b so that reverse relative rotation of the body portions 412b and 412c is resisted.

However, as will be seen, the nub 510 and guide groove 514 in cooperation with detent finger 508 serve both as a detent feature, and also serve as visual and auditory indicators of the condition of the device 440. That is, in the condition of Figure 9, the finger 508 is not aligned with the guide groove 514 and gives the impression that the trigger sleeve is not ready to move forward. On the other hand, in the condition of Figure 11, the detent finger is aligned with the guide groove 514 and it appears that the trigger sleeve can be moved forward along this guide groove (which is true). Also, as the trigger sleeve is rotated from the position of Figure 9 to that of Figure 11, the detent finger provides an audible "snap" or "click" sound as the nub 510 drops into the guide groove 514. This "snap" sound is an indication to the user of the device 410 that it is ready to effect an injection.

In order to provide for improved manual purchase or grip upon the body 412, the forward portion 412b includes a radially outer and axially extending section 412e which provides surface roughening for better manual purchase in rotating the portions 412b and 412c relative to one another. In this case, the surface roughening is provided by plural ribs or lands and grooves alternating with one another as is depicted in Figure 9. Similarly, the body 412 at aft portion 412c on trigger sleeve 422 provides a pair of diametrically opposed plateaus 412f, which are also provided with surface roughening 412g for better manual purchase. In this case, the surface roughening 412g is provided by knurling. Because of the diametrically opposed positions of the plateaus 412f on the body 412, the trigger sleeve 422 is particularly well grasped with the opposed thumb and index fingers. Thus is manual grasping and relative rotation of the body portions 412b and 412c (carrying trigger sleeve 422) effected. Also, thus is provided structure for an intuitive grasping of the device 410 by the user of the device, between the user's thumb and opposed index fingers, so that the trigger sleeve is used to position the device, and is then moved forwardly to effect an injection.

On the other hand, is it seen that the detent finger 508, recess 512, and guide groove 514 do not positively prevent reverse relative rotation of the body portions 412b and 412c from the position seen in Figure 11 back to the position seen in Figure 9. The same is true with respect to reverse relative rotation of the body portions from their positions seen in Figure 9 in a direction opposite to the arrow of this Figure (i.e., nub 510, recess 512, and guide groove 514 are merely detent, as well as visual and auditory indicator features).

Viewing Figure 10b, it is seen that in order to cooperatively permit unidirectional relative rotation of the body portions 412b and 412c from their position of Figure 9 to the position of Figure 11, the body portion 412b includes an axially extending protrusion 516, which is adjacent to a helical end surface portion 518. Recalling the description above, it will be recalled that the body portion 412b defines an end surface 462a upon which the bore 458 opens. Thus, it is seen that the helical surface portion 518 is a part of end surface 462a. The trigger sleeve 422 defines a radially inwardly and axially extending key portion 520, which includes an angulated end surface portion 520a. In the relative position of body portions 412b and 412c seen in Figure 9, the protrusion 516 engages against key portion 520 (as is illustrated in Figure 10b) so that the body portions 412b and 412c can be relatively rotated only in the direction of the arrows seen in this Figure (which is the same relative rotation illustrated by the arrows of Figure 9). In the relative position of Figure 9, the angulated end surface 520a of the key 520 bears against the helical end edge surface portion 518 so that the trigger sleeve 422 cannot be moved forwardly from the storage position seen in Figure 9. That is, the relatively large bearing area provided by the end edge surface 520a of the key 520 is sufficient to resist even attempts to manually force or jam the trigger sleeve 422 forwardly.

On the other hand, it is easily understood that the body portions 412b and 412c can be manually rotated from the relative position of Figure 9 to that of Figure 11. As this unidirectional relative rotation of the body portions 412b and 412c proceeds, the angulated end surface 520a of key 520 tracks along the helical surface 518 because this surface has the same pitch as the threads 464 and 466. In the position of Figure 11, as is illustrated by Figure 12a, the key 520 comes into contact with a circumferentially disposed step 462b on the body portion 412b. In this relative position of the body portion 412b and trigger sleeve 422, the key 520 is now in axial alignment with an axially extending keyway recess 522 defined by the body portion 412b. Thus, the trigger sleeve 422 could be moved forwardly relative to the body 412 to effect a jet injection with the injector 410.

However, in order to insure that the trigger sleeve 422 is not moved forwardly inadvertently by a user of the device 410, the device 410 includes an axial-movement resistance feature effective to prevent movement of the trigger sleeve 422 too easily in the axial forward direction, as will be further described below.

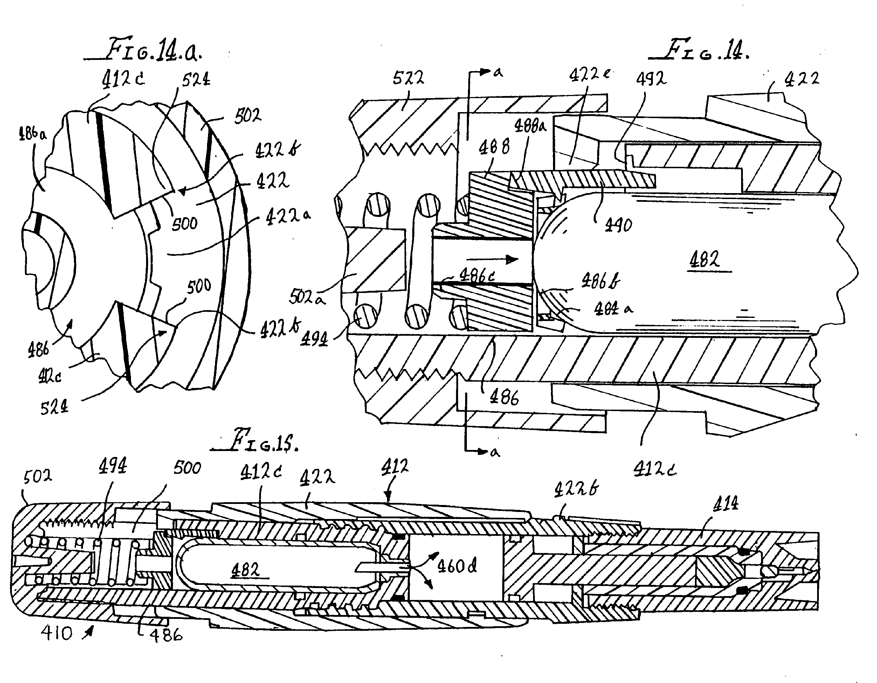

First however, in order to complete this description of the device 410, attention now to Figures 9-15, and especially Figures 13 and 14 will show that the device includes a tubular and cylindrical hammer assembly 484. This hammer assembly 484 includes a ring-like sear carrier member 484a , and a hammer/spring seat member 486 which includes an annular spring seat surface 486a, a forwardly disposed axially surface 486b engageable both with the ring like member 484a and with the pressurized gas capsule 482, and also defines three radially extending sear pocket portions 488 each defining an axially disposed sear pocket 488a. In this embodiment, the capsule 482 preferably contains a pressurized gas, and most preferably contains pressurized nitrogen gas. A seal member 482a is carried by the body portion 412, and sealingly and movably cooperates with the capsule 482.

The ring-like member 484a includes three circumferentially arrayed and axially extending sear struts 490, which in the position of the hammer assembly seen in Figure 10 rest at one end in a respective one of the sear pockets 488a. At the opposite end, each of these sear struts rests in a respective one of three catch recesses 492 inwardly defined by body portion 412c., viewing Figures 10 and 14. The sear struts 490 are pivotally connected to the ring-like member 484a by respective integral frangible living hinge sections 490a. These frangible hinge sections 490a are fractured during assembly of the device 410 so that the device is positively a single-use device. Further, the device 410 includes a spring 494 urging hammer assembly 484 forwardly. An end cap 502 captures the spring 494 and the hammer assembly 484 in the aft portion of the handpiece 412. The end cap 502 includes an axially elongate recoil buffer nose 502a, extending toward the hammer member 486. Similarly, the hammer member 486 includes a tubular recoil buffer portion 486b extending axially toward the nose portion 502a of the end cap 502.

Further, by comparing the illustration of Figure 13 with that of Figure 14, it may be seen in Figure 13 that the ring like portion 484a is disposed relatively close axially to the aft end of the struts 490. On the other hand, the axial depth of the pockets 488a on hammer member 486 is greater than the axial projection of the struts 490 aft of the ring-like portion 484a. Consequently, when the device 410 is assembled, the surface 486b of the hammer member 486 engages the ring-like member 484a well before the aft end of the struts 490 are seated completely in sear pockets 488a (although the sear members are received partially into these pockets so as to guide the sear members). However, as the end cap 502 is threaded onto body section 412c, the spring force applied via spring 494 becomes sufficient to fracture the hinge sections 490a, allowing the sear members 490 to seat completely into the pockets 488a (i.e., resulting in the relative positions of these parts seen in Figures 10 and 12).

Figure 14 illustrates the hammer and sear structure described above immediately after the moment of sear release (i.e., a split second before effecting of a hypodermic jet injection by use of the device 410). In this illustration of Figure 14, it is seen that the trigger sleeve 422 has been moved forwardly sufficiently (indicated by the axially directed arrows in Figure 14) that the trigger block portions 422a have dislodged the forward end of the sear struts 490 from their repose in catch recesses 492. Consequently, the hammer assembly 484 has started forward toward the gas capsule 482, and a jet injection will be effected when the pressurized gas within this capsule is utilized, as has been described above.

Turning now to Figure 10a, and to Figure 14a in particular, is it seen that the trigger sleeve 422 defines three radially inwardly extending trigger blocks 422a. These trigger blocks 422a are slidably received into respective axially extending slots 500 defined by the body portion 412c. As Figure 14a illustrates, the trigger blocks 422a are preferably provided with a fillet 422b at the radially outer intersection of these trigger blocks with the remainder of trigger sleeve 422. On the other hand, the adjacent portion of housing 412c (i.e., along the radially outer edge of the slots 500) is not provided with a matching "round," but instead has a comparatively sharp edge 524. This sharp edge 524 engages against the fillet 422b to frictionally resist axial movement of the trigger sleeve 422 relative to the body portion 412c until a sufficient axial force has been applied by a user of the device 410. Once this threshold value of axial force has been applied by the user of the device 410, the trigger sleeve 422 slides forward to effect a jet injection, as has been described. Most preferably, the interference relationship of the trigger blocks 422a with the fillets 422b is provided only for an initial portion of the forward firing movement of the trigger sleeve 422. After the trigger sleeve 422 is moved forwardly through this initial interference distance, the interference force requirement is discontinued, and the axial force required on the trigger sleeve 422 in order to dislodge the struts 490 then provides some resistance to the movement of the trigger sleeve until the struts 490 are dislodged, and the device 410 discharges. This threshold level of axial force required of a user on the trigger sleeve 422 in order to effect initial axial movement of this trigger sleeve insures that the trigger sleeve does not move forward inadvertently, and also insures that when the user moves this trigger sleeve forward, it is a deliberate action and is because the user is ready and intends to effect a jet injection using the device 410. Further, this selected resistance to forward motion of the trigger sleeve 422 insures that the device 410 is pressed against the skin of the person who is to receive the jet injection with a selected level of axial force. That is, the surface 416 is most preferably pressed against the skin of the recipient of the jet injection with an axial force of about 3 to 4 pounds. This level of axial force is such that it provides the optimal degree of pressure of the surface 416 on the skin of the recipient, stretching the skin just sufficiently to insure an optimal jet injection, and insuring that the surface 416 of the injector 410 maintains contact with the skin during the brief interval of the injection.

The device 410 has a condition as seen in Figure 15 for a relatively short interval during an injection, and it will be appreciated that pressurized gas from the capsule 482 (communicated to chamber 460b) is also effective to urge the capsule 482 rearwardly (leftwardly, as seen in Figure 15). Consequently, the capsule 482 will recoil leftwardly from the position seen in Figure 15 back toward its position of Figures 10 and 12. If the capsule 482 were allowed to recoil leftwardly a distance sufficient to move leftwardly of the seal 482a, then pressurized gas would be vented from the device 410. In order to prevent this venting of pressurized gas, the recoil buffer portions 486b and 502a confront and contact one another. Consequently, the capsule is stopped in its leftward recoil motion at the position of this capsule illustrated in Figures 10 and 12.

While the invention has been depicted and described by reference to two particularly preferred embodiments of the invention, its scope is defined by the appended claims.

## Claims

1. A needle-less hypodermic jet injection device comprising:
a drug injection cartridge (414) including:
a medication cylinder,
a drug-injection piston cooperating with the medication cylinder to define a variable-volume chamber,
said chamber holding a dose of substantially incompressible liquid medication, and
a fine-dimension injection orifice (418) in liquid flow communication with the variable-volume chamber to receive the liquid medication and discharge this medication as a high velocity forceful jet for hypodermic jet injection of the medication upon forceful movement of said piston in said cylinder; and
a hand piece assembly (112, 412) having a body (412a) holding said drug injection cartridge (414), said hand piece assembly (112, 412) including a hermetically sealed source of pressurized gas (182, 482) having a penetrable wall portion, and a penetrator for penetrating said penetrable wall portion of said source of pressurized gas (182, 482) and releasing pressurized gas to drive said drug-injection piston to move said drug-injection piston to cause said drug-injection piston to eject said dose of liquid medication;
**characterised in that** said injection device further comprises:
a trigger assembly for initiating forceful movement of said piston, said trigger assembly including a hammer member (184, 484) for selectively impaling said source of pressurized gas at said penetrable wall portion upon said penetrator, wherein said hammer member (184, 484) includes a plurality of axial extending legs (220, 490) configured to resist movement of said hammer member (184, 484) towards said penetrator, and a trigger sleeve (122, 422) for dislodging said axially extending legs (220, 490) so as to allow the hammer member to move said source of pressurized gas onto said penetrator, said trigger assembly further comprising a ring-like abutment member (222) or catch recesses (492), upon which end surfaces of said axial extending legs rest, said trigger sleeve (122, 422) surrounds said hammer member (184, 484) and comprises a plurality of contact portions, each engaging one of the plurality of legs to move said legs out of engagement with said one of ring-like abutment member (222) or catch recesses (492) upon axial movement of said trigger sleeve; and means (192, 494) for urging the hammer member to move the source of pressurized gas onto the penetrator

2. A needle-less hypodermic jet injection device comprising:
a drug injection cartridge (14) including:
a medication cylinder,
a drug-injection piston (50) cooperating with the medication cylinder to define a variable-volume chamber (52),
said chamber (52) holding a dose of substantially incompressible liquid medication, and
a fine-dimension injection orifice (18) in liquid flow communication with the variable-volume chamber (52) to receive the liquid medication and discharge this medication as a high velocity forceful jet (20) for hypodermic jet injection of the medication upon forceful movement of said piston (50) in said cylinder; and
a hand piece assembly (12) having a body (12a) holding said drug injection cartridge (14), said hand piece assembly (12) including a hermetically sealed source of pressurized gas (82) having a penetrable wall portion (82d), and a penetrator (78) for penetrating said penetrable wall portion of said source of pressurized gas (82) and releasing pressurized gas to drive said drug-injection piston (50) to move said drug-injection piston (50) to cause said drug-injection piston to eject said dose of liquid medication;
**characterised in that** said injection device further comprises:
a trigger assembly for initiating forceful movement of said piston (50), said trigger assembly including a hammer member (84) for selectively impaling said source of pressurized gas at said penetrable wall portion (82d) upon said penetrator (78), said hammer member having an axially extending groove (86) into which a dowel pin (88) is received, the dowel pin (88) configured to resist movement of said hammer member (84) towards said penetrator (78), and a trigger sleeve (22) for dislodging said dowel pin (88) so as to allow the hammer member to move said source of pressurized gas onto said penetrator, said trigger assembly further comprising a metal sear pin (94) on which the dowel pin (88) rests and wherein said trigger sleeve (22) surrounds said hammer member (84) and comprises a trigger block (22a) to dislodge the dowel pin (88) from the sear pin (94) upon axial movement of said trigger sleeve; and means (92) for urging the hammer member to move the source of pressurized gas onto the penetrator.

3. A jet injection device as claimed in claim 1 or claim 2, in which:
the medication cylinder has an outlet orifice (36b);
a flow path communicates the outlet orifice (36b) to said injection orifice (18, 418),
in a first position said drug-injection piston (50) cooperates with the medication cylinder to define a variable-volume chamber (52) of a first selected size;
said dose of liquid medication substantially fills said variable-volume chamber (52) at a first selected size with substantially no ullage volume,
said piston (50) has a second position cooperating with said medication cylinder to define a variable-volume chamber (52) of second selected size smaller than said first selected size;
said hand piece assembly (12, 112, 412) has a first body portion (12b, 412b) holding said drug injection cartridge (14, 414), and an abutment member (72) selectively movable into engagement with said drug-injection piston (50) to move said drug-injection piston (50) by mans of a gas power piston (60) from said first position to said second position and wherein ejecting said dose of liquid medication is caused by moving said drug-injection piston (50) from said second position to a third position.

4. A device as claimed in claim 3, in which said hand piece assembly (12) further includes a first bore (58) within said first body portion (12b), said gas power piston (60) movably received in said bore and having a ram portion (60e) extending into said drug injection cartridge (14) to abut with said drug-injection piston (50), said body and gas power piston cooperating to define a first variable-volume gas-power chamber (60d) in said first bore (58);
said hand piece assembly (12) further including a second body portion (12c) adjustably engaging with said first body portion (12b), said second body portion (12c) defining an elongate second bore (70) in gas flow communication with said first bore gas-power chamber (60d) and separated therefrom by a wall portion including said abutment member (72) carried by said second body portion (12c).

5. A device as claimed in claim 4, in which said first body portion (12b) and said second body portion (12c) are threadably and adjustably engaged with one another, said second body portion (12c) carrying said abutment member (72).

6. A device as claimed in claim 4, in which said second bore (70) in said second body portion (12c) further carries said source of pressurized gas which comprises a cylindrical gas capsule (82).

7. A device as claimed in claim 6, in which said hand piece assembly (12) second body portion (12c) is cylindrical, and said trigger sleeve is a tubular trigger sleeve (22, 122, 412) which is movably carried by said second body portion (12c) to effect opening of said gas capsule (82).

8. A device as claimed in claim 3, in which:
said medication cylinder has a plug member (44) sealingly closing the outlet orifice (36b);
the variable-volume chamber (52) of the second selected size is sufficiently smaller than said first selected size so that said plug member (44) is hydraulically forced from said outlet orifice (36b);
said hand piece assembly (12) further comprises a second body portion (12c) providing said abutment member (72);
said source of pressurized gas (82) including a hermetically sealed metallic gas capsule (82); and
said trigger assembly configured for selectively penetrating said gas capsule (82) and for applying force from said pressurized gas to said drug-injection piston (50) to move said drug-injection piston (50) from said second position to said third position.

9. A device as claimed in claim 8, in which said first body portion (12b) defines a first bore (58), said gas power piston (60) movably received in said first bore (58), said gas power piston (60) having a piston head (60a) and a ram portion (60e) extending into said drug injection cartridge (14) to abut with said drug-injection piston (50), said first body portion (12b) and said gas power piston (60) cooperating to define a first variable-volume gas-power chamber (60d) in said first bore (58);
said second body portion (12c) sealingly and movably engaging with said first body portion (12b) to bound said gas-power chamber (60d), said second body portion (12c) defining an elongate second bore (70) in gas flow communication with said gas-power chamber (60d), and said gas capsule (82) being received into said second bore (70).

10. A device as claimed in claim 1 and claim 4, in which said second bore in said second body portion (412c) further carries said source of pressurized gas comprising a cylindrical gas capsule (482) movably received in said second bore, said second body portion (412c) carrying an end cap (502) having an axially extending recoil abutment member (502a) extending toward said gas pressure capsule and effective upon recoil of said gas pressure capsule toward said end cap to limit axial motion of said gas pressure capsule toward said end cap.

11. A device as claimed in claim 1, in which:
said drug injection cartridge (414) includes an outlet orifice and a flow path communicating the outlet orifice to said injection orifice (418),
a plug member in a first position sealingly disposed in said flow path,
said drug-injection piston in a first position cooperating with said medication cylinder to define a variable-volume chamber (452) of first selected size,
said dose of liquid medication substantially filling said variable-volume chamber (452) at said first size with substantially no ullage volume,
said drug-injection piston having a second position cooperating with said medication cylinder to define a variable-volume chamber (452) of second selected size smaller than said first selected size, so that said incompressible liquid medication displaces said plug member from said first position of sealing disposition in said flow path to a second position of capture in said flow path, said medication cylinder and said plug member in said second position thereof cooperatively defining an open flow path between said variable-volume chamber (452) and said injection orifice (418);
release of said pressurised gas forcefully moves said drug-injection piston from said second position to a third position so as to reduce the volume of said variable-volume chamber (452) thereby ejecting said dose of liquid medication via said injection orifice (418); and
said hand piece assembly (412) further including a first body portion (412b) holding said drug injection cartridge (414), and an abutment member selectively movable into engagement with said drug-injection piston by means of a gas powered piston to move said drug-injection piston from said first position to said second position.

12. A device as claimed in claim 11, in which said hand piece assembly (412) further includes a first bore within said first body portion (412b), said drug-injection piston including said gas power piston (460) movably received in said bore and having a ram portion extending into said drug injection cartridge (414) to abut with a sealing member movably received therein, said body and gas power piston (460) cooperating to define a first variable-volume gas-power chamber (460d) in said first bore; said hand piece assembly (412) further including a second body portion (412c) adjustably engaging with said first body portion (412b), said second body portion (412c) defining an elongate second bore in gas flow communication with said gas-power chamber (60d) and separated therefrom by a wall portion carried by said second body portion (412c).

13. A device as claimed in claim 12, in which said second bore in said second body portion (412c) further carries a cylindrical pressurized gas capsule (482), said cylindrical gas capsule (182, 482) providing said hermetically sealed source of pressurized gas (182, 482).

14. A device as claimed in claim 12, in which said first and second body portions are threadably engaged with one another, and one of said first and second body portions includes an axially extending protrusion (516), said one of said first and second body portions also including a helical end surface portion (518) confronting the other of said first and second body portions, said trigger sleeve (422) axially movably captured on said hand piece body, and one of said first and second body portions and said trigger sleeve (422) defining a radially and axially extending key portion (520) while the other of said first and second body portions and said trigger sleeve (422) defines a radially and axially extending keyway (522), in a first relative position of said first and second body portions in which said drug-injection piston is in said first position said key portion (520) confronting said helical end surface portion (518) to prevent movement of said trigger sleeve (422) and also engaging said protrusion (516) to allow only unidirectional relative rotation of said first and second body portions, said body portions being relatively rotatable on said threaded engagement with said key portion (520) tracking said helical surface portion (518) to align with and be receivable into said keyway in a second relative position of said first and second body portions and to simultaneously move said drug-injection piston from said first to said second position by axial relative movement of said abutment member, in said second position of said first and second body portions said trigger sleeve (422) being movable to receive said key portion (520) into said keyway (522) and to effect
relase of said pressurised gas.

15. A device as claimed in claim 1 or claim 2, in which:
said drug-injection piston is movable from a first to a second position, said drug-injection piston being cooperable with said drug injection cartridge (14, 414) to forcefully reduce the volume of said medication cylinder for displacing said liquid medication as said high velocity injection jet; and
said drug-injection piston (50) being reciprocally held by said hand piece assembly (12, 412), release of said pressurised gas forcefully moving said drug-injection piston (50) selectively from said first to said second position; said hand piece body including first and second body portions (12c) threadably engaged with one another, and one of said first and second body portions (12c) includes an axially extending protrusion confronting the other of said first and second body portions (12c), said one of said first and second body portions (12c) also including a helical end surface portion extending circumferentially from said protrusion and also confronting the other of said first and second body portions (12c), said trigger sleeve axially movable on said hand piece body, and one of said first and second body portions (12c) and said trigger sleeve (22, 122) defining a radially and axially extending key portion while the other of said first and second body portions (12c) and said trigger sleeve (22, 122) defines a radially and axially extending keyway, in a first relative position of said first and second body portions (12c) said key portion confronting said helical end surface portion to prevent movement of said trigger sleeve and also engaging said protrusion to allow only unidirectional relative rotation of said first and second body portions (12c), said body portions being relatively rotatable on said threaded engagement with said key portion tracking said helical surface portion to align with and be receivable into said keyway in a second relative position of said first and second body portions (12c), in said second relative position of said first and second body portions (12c) said trigger sleeve being movable to receive said key portion into said keyway and to effect release of said pressurised gas. .

16. A device as claimed in claim 15, further including a first bore (58) within said first body portion (12b), said drug-injection piston (50) including a gas power piston (60) movably received in said bore and having a ram portion (54) extending into said drug injection cartridge (14) to abut with a sealing member movably received therein, said body and gas power piston (60) cooperating to define a first variable-volume gas-power chamber (60d) in said first bore (58).

17. A device as claimed in claim 1 wherein:
said hand piece assembly includes a first body portion, and a second body portion rotational relative to one another;
one of said first body portion and said second body portion defining a detent finger (508) having a radial projection (510) disposed proximate to a distal end (508a) thereof, and the other of said first and second body portions including a radially outer surface across which said distal end (508a) sweeps in response to relative rotation of said body portions; and
in a first relative position of said body portions, the projection (510) from said detent finger (508) being received into a recess (512) defined on said radially outer surface, and in response to relative rotation of said body portions, said detent finger distal end (508a) sweeping across said radially outer surface to drop the projection (510) radially into a second recess (514) on said surface, whereby the dropping of said projection (510) into said second recess (514) provides both a visual and an auditory indication of said body portions being in said second relative position.

18. A device as claimed in claim 1 wherein:
said hand piece assembly (412) includes a first body portion (412b), and a second body portion (412c) rotational relative to one another;
a forward one of said first body portion (412b) and said second body portion (412c) defining a radially outer axially extending surface for providing manual purchase upon said device, said surface including surface roughening for improving manual purchase;
a rearward one of said first body portion (412b) and said second body portion (412c) defining a radially outer surface having a pair of diametrically opposite radially extending plateaus each provided with surface roughening for improving manual purchase, particularly of opposed index and thumb fingers.

19. A device as claimed in claim 1 wherein said source of pressurized gas includes a gas capsule (482) for impaling upon said penetrator to release pressurized gas from within said gas capsule (482), in response to said hammer member (484) effecting axial driving of said gas capsule (482) onto said penetrator, said hammer member (484) comprising:
a ring-like sear carrier member (484a),
a plurality of sear struts (490) integral with said sear carrier member (484a); and
a corresponding plurality of integral frangible hinge sections (490a) each connecting a respective one of said plurality of sear struts with said sear carrier member (484a).

20. A device as claimed in claim 1, wherein said hammer member (184) comprises a unitary elongate molded plastic polymer hammer member (184) comprising:
a central wall portion (218) extending radially;
a circular cylindrical portion (216) extending axially in one direction from said central wall portion, said circular cylindrical portion including a tubular skirt and cooperating with said central wall portion to define a spring seat (216a) into which a spring (192) may be received to engage upon said central wall portion;
a conically flaring portion (220a) extending in an opposite axial direction from said central wall portion, said conical portion including a plurality of circumferentially spaced apart resilient legs (220) each extending axially to terminate in a respective axial end surface (220b) engageable with said ring like abutment member (222) in a first position of the legs (220) to support said hammer member (184) in opposition to force from the spring (192) exerted on said central wall portion (218), said legs in said first position cooperatively defining a conical diameter at said end surfaces, and said legs at said end surfaces each also defining a circular radius which are substantially equal to the radius of said circular cylindrical portion;
whereby, said plurality of legs (220) are movable to a second position in opposition to said resilience of said legs, in said second position said plurality of legs being circumferentially nested adjacent to one another and cooperatively defining a diameter substantially equal to that of said cylindrical portion.

## Patentansprüche

1. Nadellose subkutane Injektionsvorrichtung, die aufweist:
eine Arzneimittelinjektionspatrone (414), die umfasst:
einen Medikationszylinder;
einen Arzneimittelinjektionskolben, der mit dem Medikationszylinder zusammenwirkt, um eine regelbare Kammer zu definieren,
wobei die Kammer eine Dosis der im Wesentlichen nicht komprimierbaren flüssigen Medikation aufnimmt; und
eine Injektionsöffnung (418) mit genauer Abmessung in Flüssigkeitsströmungsverbindung mit der regelbaren Kammer, um die flüssige Medikation aufzunehmen und diese Medikation als einen wirksamen Strahl mit hoher Geschwindigkeit für die subkutane Injektion der Medikation bei einer wirksamen Bewegung des Kolbens im Zylinder auszustoßen; und
eine Handstückbaugruppe (112, 412) mit einem Gehäuse (412a), die die Arzneimittelinjektionspatrone (414) aufnimmt, wobei die Handbaugruppe (112, 412) eine hermetisch abgedichtete Druckgasquelle (182, 482) mit einem durchdringbaren Wandabschnitt und einen Eindringkörper für das Durchdringen des durchdringbaren Wandabschnittes der Druckgasquelle (182, 482) und das Freigeben des Druckgases umfasst, um den Arzneimittelinjektionskolben anzutreiben, um den Arzneimittelinjektionskolben zu bewegen, um zu veranlassen, dass der Arzneimittelinjektionskolben die Dosis der flüssigen Medikation ausstößt;
**dadurch gekennzeichnet, dass** die Injektionsvorrichtung außerdem aufweist:
eine Auslöserbaugruppe für das Einleiten einer wirksamen Bewegung des Kolbens, wobei die Auslöserbaugruppe umfasst: ein Schlagelement (184, 484) für das selektive Durchbohren der Druckgasquelle im durchdringbaren Wandabschnitt mittels des Eindringkörpers, wobei das Schlagelement (184, 484) eine Vielzahl von sich axial erstreckenden Schenkeln (220, 490) umfasst, die ausgebildet sind, um der Bewegung des Schlagelementes (184, 484) in Richtung des Eindringkörpers zu widerstehen; und eine Auslöserhülse (122, 422) für das Verrücken der sich axial erstreckenden Schenkel (220, 490), um so zu gestatten, dass das Schlagelement die Druckgasquelle auf den Eindringkörper bewegt, wobei die Auslöserbaugruppe außerdem aufweist: ein ringartiges Anschlagelement (222) oder Auffangaussparungen (492), auf denen die Endflächen der sich axial erstreckenden Schenkel aufliegen, wobei die Auslöserhülse (122, 422) das Schlagelement (184, 484) umgibt und eine Vielzahl von Kontaktabschnitten aufweist, von denen ein jeder mit einem der Vielzahl der Schenkel in Eingriff kommt, um die Schenkel außer Eingriff mit dem einen von ringartigem Anschlagelement (222) oder Auffangaussparungen (492) bei einer axialen Bewegung der Auslöserhülse zu bringen; und eine Einrichtung (192, 494) für das Treiben des Schlagelementes, um die Druckgasquelle auf den Eindringkörper zu bewegen.

2. Nadellose subkutane Injektionsvorrichtung, die aufweist:
eine Arzneimittelinjektionspatrone (14), die umfasst:
einen Medikationszylinder;
einen Arzneimittelinjektionskolben (50), der mit dem Medikationszylinder zusammenwirkt, um eine regelbare Kammer (52) zu definieren,
wobei die Kammer (52) eine Dosis der im Wesentlichen nicht komprimierbaren flüssigen Medikation aufnimmt; und
eine Injektionsöffnung (18) mit genauer Abmessung in Flüssigkeitsströmungsverbindung mit der regelbaren Kammer (52), um die flüssige Medikation aufzunehmen und diese Medikation als einen wirksamen Strahl (20) mit hoher Geschwindigkeit für die subkutane Injektion der Medikation bei einer wirksamen Bewegung des Kolbens (50) im Zylinder auszustoßen; und
eine Handstückbaugruppe (12) mit einem Gehäuse (12a), die die Arzneimittelinjektionspatrone (14) aufnimmt, wobei die Handbaugruppe (12) eine hermetisch abgedichtete Druckgasquelle (82) mit einem durchdringbaren Wandabschnitt (82d) und einen Eindringkörper (78) für das Durchdringen des durchdringbaren Wandabschnittes der Druckgasquelle (82) und das Freigeben des Druckgases umfasst, um den Arzneimittelinjektionskolben (50) anzutreiben, um den Arzneimittelinjektionskolben (50) zu bewegen, um zu veranlassen, dass der Arzneimittelinjektionskolben die Dosis der flüssigen Medikation ausstößt;
**dadurch gekennzeichnet, dass** die Injektionsvorrichtung außerdem aufweist:
eine Auslöserbaugruppe für das Einleiten einer wirksamen Bewegung des Kolbens (50), wobei die Auslöserbaugruppe umfasst: ein Schlagelement (84) für das selektive Durchbohren der Druckgasquelle im durchdringbaren Wandabschnitt (82d) mittels des Eindringkörpers (78), wobei das Schlagelement eine sich axial erstreckende Nut (86) aufweist, in der ein Passstift (88) aufgenommen wird, wobei der Passstift (88) ausgebildet ist, um der Bewegung des Schlagelementes (84) in Richtung des Eindringkörpers (78) zu widerstehen; und eine Auslöserhülse (22) für das Verrücken des Passstiftes (88), um so zu gestatten, dass das Schlagelement die Druckgasquelle auf den Eindringkörper bewegt, wobei die Auslöserbaugruppe außerdem aufweist: einen Metallabzugshebelstift (94), auf dem der Passstift (88) aufliegt, und wobei die Auslöserhülse (22) das Schlagelement (84) umgibt und einen Auslöserblock (22a) aufweist, um den Passstift (88) vom Abzugshebelstift (94) bei einer axialen Bewegung der Auslöserhülse zu verrücken; und eine Einrichtung (92) für das Treiben des Schlagelementes, um die Druckgasquelle auf den Eindringkörper zu bewegen.

3. Injektionsvorrichtung nach Anspruch 1 oder Anspruch 2, bei der:
der Medikationszylinder eine Austrittsöffnung (36b) aufweist;
ein Strömungsweg die Austrittsöffnung (36b) mit der Injektionsöffnung (18, 418) verbindet;
in einer ersten Position der Arzneimittelinjektionskolben (50) mit dem Medikationszylinder zusammenwirkt, um eine regelbare Kammer (52) mit einer ersten ausgewählten Größe zu definieren;
die Dosis der flüssigen Medikation im Wesentlichen die regelbare Kammer (52) mit einer ersten ausgewählten Größe bei im Wesentlichen keinem Volumenschwund füllt;
der Kolben (50) eine zweite Position aufweist, die mit dem Medikationszylinder zusammenwirkt, um eine regelbare Kammer (52) mit einer zweiten ausgewählten Größe zu definieren, die kleiner ist als die erste ausgewählte Größe;
die Handstückbaugruppe (12, 112, 412) einen ersten Gehäuseabschnitt (12b, 412b), der die Arzneimittelinjektionspatrone (14, 414) aufnimmt, und ein Anschlagelement (72) aufweist, das selektiv in Eingriff mit dem Arzneimittelinjektionskolben (50) beweglich ist, um den Arzneimittelinjektionskolben (50) mittels eines Gasarbeitskolbens (60) aus der ersten Position in die zweite Position zu bewegen,
und wobei das Ausstoßen der Dosis der flüssigen Medikation durch die Bewegung des Arzneimittelinjektionskolbens (50) aus der zweiten Position in eine dritte Position bewirkt wird.

4. Vorrichtung nach Anspruch 3, bei der die Handstückbaugruppe (12) außerdem eine erste Bohrung (58) innerhalb des ersten Gehäuseabschnittes (12b) umfasst, wobei der Gasarbeitskolben (60) beweglich in der Bohrung aufgenommen wird und einen Stößelabschnitt (60e) aufweist, der sich in die Arzneimittelinjektionspatrone (14) erstreckt, um an den Arzneimittelinjektionskolben (50) anzustoßen, wobei das Gehäuse und der Gasarbeitskolben zusammenwirken, um eine erste regelbare Gasarbeitskammer (60d) in der ersten Bohrung (58) zu definieren;
wobei die Handstückbaugruppe (12) außerdem einen zweiten Gehäuseabschnitt (12c) umfasst, der verstellbar mit dem ersten Gehäuseabschnitt (12b) in Eingriff gebracht wird, wobei der zweite Gehäuseabschnitt (12c) eine längliche zweite Bohrung (70) in Gasströmungsverbindung mit der Gasarbeitskammer (60d) in der ersten Bohrung definiert und davon mittels eines Wandabschnittes getrennt ist, der das Anschlagelement (72) umfasst, das vom zweiten Gehäuseabschnitt (12c) aufgenommen wird.

5. Vorrichtung nach Anspruch 4, bei der der erste Gehäuseabschnitt (12b) und der zweite Gehäuseabschnitt (12c) verschraubbar und verstellbar miteinander in Eingriff gebracht werden, wobei der zweite Gehäuseabschnitt (12c) das Anschlagelement (72) aufnimmt.

6. Vorrichtung nach Anspruch 4, bei der die zweite Bohrung (70) im zweiten Gehäuseabschnitt (12c) außerdem die Druckgasquelle aufnimmt, die eine zylindrische Gaskapsel (82) aufweist.

7. Vorrichtung nach Anspruch 6, bei der der zweite Gehäuseabschnitt (12c) der Handstückbaugruppe (12) zylindrisch ist, und wobei die Auslöserhülse eine rohrförmige Auslöserhülse (22, 122, 412) ist, die vom zweiten Gehäuseabschnitt (12c) beweglich getragen wird, um das Öffnen der Gaskapsel (82) zu bewirken.

8. Vorrichtung nach Anspruch 3, bei der:
der Medikationszylinder ein Stopfenelement (44) aufweist, das abdichtend die Austrittsöffnung (36b) verschließt;
die regelbare Kammer (52) mit der zweiten ausgewählten Größe ausreichend kleiner ist als die erste ausgewählte Größe, so dass das Stopfenelement (44) hydraulisch aus der Austrittsöffnung (36b) gedrückt wird;
die Handstückbaugruppe (12) außerdem einen zweiten Gehäuseabschnitt (12c) aufweist, der das Anschlagelement (72) bereitstellt;
die Druckgasquelle (82) eine hermetisch abgedichtete Metallgaskapsel (82) umfasst; und
die Auslöserbaugruppe für ein selektives Durchdringen der Gaskapsel (82) und für das Anwenden einer Kraft vom Druckgas auf den Arzneimittelinjektionskolben (50) ausgebildet ist, um den Arzneimittelinjektionskolben (50) aus der zweiten Position in die dritte Position zu bewegen.

9. Vorrichtung nach Anspruch 8, bei der der erste Gehäuseabschnitt (12b) eine erste Bohrung (58) definiert, wobei der Gasarbeitskolben (60) beweglich in der ersten Bohrung (58) aufgenommen wird, wobei der Gasarbeitskolben (60) einen Kolbenkopf (60a) und einen Stößelabschnitt (60e) aufweist, der sich in die Arzneimittelinjektionspatrone (14) erstreckt, um an den Arzneimittelinjektionskolben (50) anzustoßen, wobei der erste Gehäuseabschnitt (12b) und der Gasarbeitskolben (60) zusammenwirken, um eine erste regelbare Gasarbeitskammer (60d) in der ersten Bohrung (58) zu definieren;
wobei der zweite Gehäuseabschnitt (12c) abdichtend und beweglich mit dem ersten Gehäuseabschnitt (12b) in Eingriff kommt, um die Gasarbeitskammer (60d) zu begrenzen, wobei der zweite Gehäuseabschnitt (12c) eine längliche zweite Bohrung (70) in Gasströmungsverbindung mit der Gasarbeitskammer (60d) definiert, und wobei die Gaskapsel (82) in der zweiten Bohrung (70) aufgenommen wird.

10. Vorrichtung nach Anspruch 1 und Anspruch 4, bei der die zweite Bohrung im zweiten Gehäuseabschnitt (412c) außerdem die Druckgasquelle aufnimmt, die eine zylindrische Gaskapsel (482) aufweist, die beweglich in der zweiten Bohrung aufgenommen wird, wobei der zweite Gehäuseabschnitt (412c) eine Abschlusskappe (502) trägt, die ein sich axial erstreckendes Rückstoßanschlagelement (502a) aufweist, das sich in Richtung der Gasdruckkapsel erstreckt und beim Rückschlag der Gasdruckkapsel in Richtung der Abschlusskappe wirksam ist, um die axiale Bewegung der Gasdruckkapsel in Richtung der Abschlusskappe zu begrenzen.

11. Vorrichtung nach Anspruch 1, bei der:
die Arzneimittelinjektionspatrone (414) eine Austrittsöffnung und einen Strömungsweg umfasst, der die Austrittsöffnung mit der Injektionsöffnung (418) verbindet;
ein Stopfenelement in einer ersten Position, das abdichtend im Strömungsweg angeordnet ist;
wobei der Arzneimittelinjektionskolben in einer ersten Position mit dem Medikationszylinder zusammenwirkt, um eine regelbare Kammer (452) mit einer ersten ausgewählten Größe zu definieren;
wobei die Dosis der flüssigen Medikation im Wesentlichen die regelbare Kammer (452) bei der ersten Größe mit im Wesentlichen keinem Volumenschwund füllt;
wobei der Arzneimittelinjektionskolben, der eine zweite Position aufweist, mit dem Medikationszylinder zusammenwirkt, um eine regelbare Kammer (452) mit einer zweiten ausgewählten Größe kleiner als die erste ausgewählte Größe zu definieren, so dass die nicht komprimierbare flüssige Medikation das Stopfenelement aus der ersten Position der abdichtenden Anordnung im Strömungsweg in eine zweite Position verschiebt, in der ein Erfassen im Strömungsweg erfolgt, wobei der Medikationszylinder und das Stopfenelement in dessen zweiter Position zusammenwirkend einen offenen Strömungsweg zwischen der regelbaren Kammer (452) und der Injektionsöffnung (418) definieren;
die Freigabe des Druckgases den Arzneimittelinjektionskolben wirksam aus der zweiten Position in eine dritte Position bewegt, um so das Volumen der regelbaren Kammer (452) zu verringern, wodurch die Dosis der flüssigen Medikation mittels der Injektionsöffnung (418) ausgestoßen wird; und
die Handstückbaugruppe (412) außerdem einen ersten Gehäuseabschnitt (412b), der die Arzneimittelinjektionspatrone (414) aufnimmt, und ein Anschlagelement umfasst, das selektiv in Eingriff mit dem Arzneimittelinjektionskolben mittels eines Gasarbeitskolbens beweglich ist, um den Arzneimittelinjektionskolben aus der ersten Position in die zweite Position zu bewegen.

12. Vorrichtung nach Anspruch 11, bei der die Handstückbaugruppe (412) außerdem eine erste Bohrung innerhalb des ersten Gehäuseabschnittes (412b) umfasst, wobei der Arzneimittelinjektionskolben den Gasarbeitskolben (460) umfasst, der beweglich in der Bohrung aufgenommen wird und einen Stößelabschnitt aufweist, der sich in die Arzneimittelinjektionspatrone (414) erstreckt, um an ein Dichtungselement anzustoßen, das darin beweglich aufgenommen wird, wobei das Gehäuse und der Gasarbeitskolben (460) zusammenwirken, um eine erste regelbare Gasarbeitskammer (460d) in der ersten Bohrung zu definieren, wobei die Handstückbaugruppe (412) außerdem einen zweiten Gehäuseabschnitt (412c) umfasst, der verstellbar mit dem ersten Gehäuseabschnitt (412b) in Eingriff kommt, wobei der zweite Gehäuseabschnitt (412c) eine längliche zweite Bohrung in einer Gasströmungsverbindung mit der Gasarbeitskammer (60d) definiert und davon durch einen Wandabschnitt getrennt ist, der vom zweiten Gehäuseabschnitt (412c) getragen wird.

13. Vorrichtung nach Anspruch 12, bei der die zweite Bohrung im zweiten Gehäuseabschnitt (412c) außerdem eine zylindrische Druckgaskapsel (482) aufnimmt, wobei die zylindrische Gaskapsel (182, 482) die hermetisch abgedichtete Druckgasquelle (182, 482) bereitstellt.

14. Vorrichtung nach Anspruch 12, bei der der erste und zweite Gehäuseabschnitt verschraubbar miteinander in Eingriff gebracht werden, und bei der einer von erstem und zweitem Gehäuseabschnitt einen sich axial erstreckenden Vorsprung (516) umfasst, wobei der eine von erstem und zweitem Gehäuseabschnitt ebenfalls einen schraubenförmigen Stirnflächenabschnitt (518) umfasst, der dem anderen von erstem und zweitem Gehäuseabschnitt gegenüberliegt, wobei die Auslöserhülse (422) axial beweglich auf dem Handstückgehäuse erfasst wird, und wobei einer von erstem und zweitem Gehäuseabschnitt und die Auslöserhülse (422) einen sich radial und axial erstreckenden Keilabschnitt (520) definieren, während der andere von erstem und zweitem Gehäuseabschnitt und die Auslöserhülse (422) eine sich radial und axial erstreckende Keilnut (522) definieren, wobei in einer ersten relativen Position des ersten und zweiten Gehäuseabschnittes, in der sich der Arzneimittelinjektionskolben in der ersten Position befindet, der Keilabschnitt (520) dem schraubenförmigen Stirnflächenabschnitt (518) gegenüberliegt, um eine Bewegung der Auslöserhülse (422) zu verhindern, und um ebenfalls mit dem Vorsprung (516) in Eingriff zu kommen, um nur eine einseitig gerichtete relative Drehung des ersten und zweiten Gehäuseabschnittes zu gestatten, wobei die Gehäuseabschnitte beim Gewindeeingriff mit dem Keilabschnitt (520) relativ drehbar sind, dem schraubenförmigen Flächenabschnitt (518) folgend, um sich mit der Keilnut in einer zweiten relativen Position des ersten und zweiten Gehäuseabschnittes auszurichten und darin aufnehmbar zu sein, und um gleichzeitig den Arzneimittelinjektionskolben aus der ersten in die zweite Position mittels einer axialen relativen Bewegung des Anschlagelementes zu bewegen, wobei in der zweiten Position des ersten und zweiten Gehäuseabschnittes die Auslöserhülse (422) beweglich ist, um den Keilabschnitt (520) in der Keilnut (522) aufzunehmen, und um die Freigabe des Druckgases zu bewirken.

15. Vorrichtung nach Anspruch 1 oder Anspruch 2, bei der:
der Arzneimittelinjektionskolben aus einer ersten in eine zweite Position beweglich ist, wobei der Arzneimittelinjektionskolben mit der Arzneimittelinjektionspatrone (14, 414) zusammenwirken kann, um das Volumen des Medikationszylinders für das Verdrängen der flüssigen Medikation als Injektionsstrahl mit hoher Geschwindigkeit wirksam zu verringern; und
wobei der Arzneimittelinjektionskolben (50) hin- und hergehend durch die Handstückbaugruppe (12, 412) gehalten wird, wobei die Freigabe des Druckgases den Arzneimittelinjektionskolben (50) selektiv von der ersten in die zweite Position wirksam bewegt; wobei das Handstückgehäuse einen ersten und zweiten Gehäuseabschnitt (12c) umfasst, die verschraubbar miteinander in Eingriff gebracht werden, und wobei einer von erstem und zweitem Gehäuseabschnitt (12c) einen sich axial erstreckenden Vorsprung umfasst, der dem anderen von erstem und zweitem Gehäuseabschnitt (12c) gegenüberliegt, wobei der eine von erstem und zweitem Gehäuseabschnitt (12c) ebenfalls einen schraubenförmigen Stirnflächenabschnitt umfasst, der sich peripher vom Vorsprung erstreckt und ebenfalls dem anderen von erstem und zweitem Gehäuseabschnitt (12c) gegenüberliegt, wobei die Auslöserhülse axial auf dem Handstückgehäuse beweglich ist, und wobei einer von erstem und zweitem Gehäuseabschnitt (12c) und die Auslöserhülse (22, 122) einen sich radial und axial erstreckenden Keilabschnitt definieren, während der andere von erstem und zweitem Gehäuseabschnitt (12c) und die Auslöserhülse (22, 122) eine sich radial und axial erstreckende Keilnut definiert, wobei in einer ersten relativen Position des ersten und zweiten Gehäuseabschnittes (12c) der Keilabschnitt dem schraubenförmigen Stirnflächenabschnitt gegenüberliegt, um eine Bewegung der Auslöserhülse zu verhindern, und um ebenfalls mit dem Vorsprung in Eingriff zu kommen, um nur eine einseitig gerichtete relative Drehung des ersten und zweiten Gehäuseabschnittes (12c) zu gestatten, wobei die Gehäuseabschnitte beim Gewindeeingriff mit dem Keilabschnitt relativ drehbar sind, dem schraubenförmigen Flächenabschnitt folgend, um sich mit der Keilnut in einer zweiten relativen Position des ersten und zweiten Gehäuseabschnittes (12c) auszurichten und darin aufnehmbar zu sein, wobei in der zweiten relativen Position des ersten und zweiten Gehäuseabschnittes (12c) die Auslöserhülse beweglich ist, um den Keilabschnitt in der Keilnut aufzunehmen, und um die Freigabe des Druckgases zu bewirken.

16. Vorrichtung nach Anspruch 15, die außerdem eine erste Bohrung (58) innerhalb des ersten Gehäuseabschnittes (12b) umfasst, wobei der Arzneimittelinjektionskolben (50) einen Gasarbeitskolben (60) umfasst, der in der Bohrung beweglich aufgenommen wird und einen Stößelabschnitt (54) aufweist, der sich in die Arzneimittelinjektionspatrone (14) erstreckt, um an ein darin beweglich aufgenommenes Dichtungselement anzustoßen, wobei das Gehäuse und der Gasarbeitskolben zusammenwirken, um eine erste regelbare Gasarbeitskammer (60d) in der ersten Bohrung (58) zu definieren.

17. Vorrichtung nach Anspruch 1, bei der:
die Handstückbaugruppe einen ersten Gehäuseabschnitt und einen zweiten Gehäuseabschnitt umfasst, die relativ zueinander drehbar sind;
einer von erstem Gehäuseabschnitt und zweitem Gehäuseabschnitt einen Arretierfinger (508) mit einem radialen Vorsprung (510) definiert, der in unmittelbarer Nähe zu einem distalen Ende (508a) davon angeordnet ist, und wobei der andere des ersten und zweiten Gehäuseabschnittes eine radial äußere Fläche umfasst, über die das distale Ende (508a) als Reaktion auf die relative Drehung der Gehäuseabschnitte schwenkt; und
in einer ersten relativen Position der Gehäuseabschnitte der Vorsprung (510) vom Arretierfinger (508) in einer Vertiefung (512) aufgenommen wird, die in der radial äußeren Fläche definiert wird, und wobei als Reaktion auf die relative Drehung der Gehäuseabschnitte das distale Ende (508a) des Arretierfingers über die radial äußere Fläche schwenkt, um den Vorsprung (510) radial in eine zweite Vertiefung (514) in der Fläche fallen zu lassen, wobei das Fallenlassen des Vorsprunges (510) in die zweite Vertiefung (514) sowohl eine visuelle als auch eine hörbare Anzeige bewirkt, dass sich die Gehäuseabschnitte in der zweiten relativen Position befinden.

18. Vorrichtung nach Anspruch 1, bei der:
die Handstückbaugruppe (412) einen ersten Gehäuseabschnitt (412b) und einen zweiten Gehäuseabschnitt (412c) umfasst, die relativ zueinander drehbar sind;
ein vorderer von erstem Gehäuseabschnitt (412b) und zweitem Gehäuseabschnitt (412c) eine radial äußere sich axial erstreckende Fläche für das Bewirken eines manuellen Hebens auf die Vorrichtung definiert, wobei die Fläche eine Oberflächenaufrauung für das Verbessern des manuellen Hebens umfasst;
ein hinterer von erstem Gehäuseabschnitt (412b) und zweitem Gehäuseabschnitt (412c) eine radial äußere Fläche mit einem Paar von diametral entgegengesetzten sich radial erstreckenden Plateaus definiert, die jeweils mit einer Oberflächenaufrauung für das Verbessern des manuellen Hebens versehen sind, insbesondere für die gegenüberliegenden Zeigefinger und Daumen.

19. Vorrichtung nach Anspruch 1, bei der die Druckgasquelle eine Gaskapsel (482) für ein Durchbohren auf dem Eindringkörper umfasst, um Druckgas aus der Gaskapsel (482) als Reaktion darauf freizugeben, dass das Schlagelement (484) ein axiales Treiben der Gaskapsel (482) auf den Eindringkörper bewirkt, wobei das Schlagelement (484) aufweist:
ein ringförmiges Abzugshebelträgerelement (484a);
eine Vielzahl von Abzugshebelstreben (490) zusammenhängend mit dem Abzugshebelträgerelement (484a); und
eine entsprechende Vielzahl von integrierten zerbrechlichen Gelenkabschnitten (490a), von denen ein jeder eine entsprechende der Vielzahl von Abzugshebelstreben mit dem Abzugshebelträgerelement (484a) verbindet.

20. Vorrichtung nach Anspruch 1, bei der das Schlagelement (184) ein vereinheitlichtes längliches geformtes Schlagelement (184) aus Kunststoffpolymer aufweist, das aufweist:
einen mittleren Wandabschnitt (218), der sich radial erstreckt;
einen kreisförmigen zylindrischen Abschnitt (216), der sich axial in einer Richtung vom mittleren Wandabschnitt erstreckt, wobei der kreisförmige zylindrische Abschnitt einen rohrförmigen Rand umfasst und mit dem mittleren Wandabschnitt zusammenwirkt, um einen Federsitz (216a) zu definieren, in dem eine Feder (192) aufgenommen werden kann, um mit dem mittleren Wandabschnitt in Eingriff zu kommen;
einen sich konisch aufweitenden Abschnitt (220a), der sich in einer entgegengesetzten axialen Richtung vom mittleren Wandabschnitt aus erstreckt, wobei der konische Abschnitt eine Vielzahl von peripher beabstandeten elastischen Schenkeln (220) umfasst, von denen sich ein jeder axial erstreckt, um in einer jeweiligen axialen Stirnfläche (220b) zu enden, die mit dem ringartigen Anschlagelement (222) in einer ersten Position der Schenkel (220) in Eingriff kommen kann, um das Schlagelement (184) entgegen der Kraft von der Feder (192) zu stützen, die auf den mittleren Wandabschnitt (218) ausgeübt wird, wobei die Schenkel in der ersten Position zusammenwirkend einen konischen Durchmesser an den Stirnflächen definieren, und wobei die Schenkel an den Stirnflächen ebenfalls jeweils einen Kreisradius definieren, die im Wesentlichen gleich dem Radius des kreisförmigen zylindrischen Abschnittes sind;
wodurch die Vielzahl der Schenkel (220) in eine zweite Position entgegen der Elastizität der Schenkel beweglich ist, wobei in der zweiten Position die Vielzahl der Schenkel peripher benachbart zueinander ineinandergesteckt ist und zusammenwirkend einen Durchmesser definiert, der im Wesentlichen gleich dem des zylindrischen Abschnittes ist.

## Revendications

1. Dispositif d'injection hypodermique par jet sans aiguille, comprenant :
une cartouche d'injection du médicament (414), englobant :
un cylindre à médicament ;
un piston d'injection du médicament, coopérant avec le cylindre à médicament pour définir une chambre à volume variable ;
ladite chambre contenant une dose de médicament liquide pratiquement incompressible ; et
un orifice d'injection à dimension fine (418), en communication d'écoulement de liquide avec la chambre à volume variable pour recevoir le médicament liquide et décharger ce médicament sous forme d'un jet sous forte pression et à vitesse élevée, en vue d'une injection hypodermique par jet du médicament lors du déplacement sous forte pression dudit piston dans ledit cylindre ; et
un assemblage de pièce à main (112 , 412), comportant un corps (412a) contenant ladite cartouche d'injection de médicament (414), ledit assemblage de pièce à main (112, 412) englobant une source de gaz sous pression hermétiquement close (182, 482), comportant une partie de paroi pénétrable et un pénétrateur pour pénétrer à travers ladite partie de paroi pénétrable de ladite source de gaz sous pression (182, 482) et libérer le gaz sous pression pour entraîner ledit piston d'injection du médicament, afin de déplacer ledit piston d'injection du médicament, pour entraîner ledit piston d'injection du médicament à éjecter ladite dose de médicament liquide ;
**caractérisé en ce que** ledit dispositif d'injection comprend en outre :
un assemblage de déclenchement pour initialiser de déplacement sous forte pression dudit piston, ledit assemblage de déclenchement englobant un élément de percuteur (184, 484) pour pénétrer sélectivement à travers ladite source de gaz sous pression au niveau de ladite partie de paroi pénétrable au moyen dudit pénétrateur, ledit élément de percuteur (184, 484) englobant plusieurs branches à extension axiale (220, 490), configurées de sorte à résister au déplacement dudit élément de percuteur (184, 484) vers ledit pénétrateur, et une douille de déclenchement (122, 422) pour déplacer lesdites branches à extension axiale (220, 490), pour permettre à l'élément de percuteur de déplacer ladite source de gaz sous pression sur ledit pénétrateur, ledit assemblage de déclenchement comprenant en outre un élément de butée de type annulaire (222) ou des évidements d'arrêt (492), sur lesquels reposent les surfaces d'extrémité desdites branches à extension axiale, ladite douille de déclenchement (122 , 422) entourant ledit élément de percuteur (184, 484) et comprenant plusieurs parties de contact, s'engageant chacune dans une des plusieurs branches pour déplacer lesdites branches et les dégager dudit un élément des éléments de butée de type annulaire (222) ou des évidements d'arrêt (492) lors du déplacement axial de ladite douille de déclenchement, et un moyen (192, 494) pour pousser l'élément de percuteur afin de déplacer la source de gaz sous pression sur le pénétrateur.

2. Dispositif d'injection hypodermique par jet sans aiguille, englobant :
une cartouche d'injection d'un médicament (14), englobant :
un cylindre à médicament ;
un piston d'injection du médicament (50), coopérant avec le cylindre à médicament pour définir une chambre à volume variable (52) ;
ladite chambre (52) contenant une dose de médicament liquide pratiquement incompressible ; et
un orifice d'injection à dimension fine (18), en communication d'écoulement de liquide avec la chambre à volume variable (52), pour recevoir le médicament liquide et décharger ce médicament sous forme d'un jet sous forte pression et à vitesse élevée (20), en vue d'une injection hypodermique par jet du médicament lors du déplacement sous forte pression dudit piston (50) dans ledit cylindre ; et
un assemblage de pièce à main (12), comportant un corps (12a) contenant ladite cartouche d'injection du médicament (14), ledit assemblage de pièce à main englobant une source de gaz sous pression fermée de manière hermétique (82), comportant une partie de paroi pénétrable (82d) et un pénétrateur (78) pour pénétrer dans ladite partie de paroi pénétrable de ladite source de gaz sous pression (82) et libérer le gaz sous pression pour entraîner ledit piston d'injection du médicament (50), afin de déplacer ledit piston d'injection du médicament (50) pour entraîner ledit piston d'injection du médicament à éjecter ladite dose de médicament liquide ;
**caractérisé en ce que** ledit dispositif d'injection comprend en outre :
un assemblage de déclenchement pour initialiser le déplacement sous forte pression dudit piston (50), ledit assemblage de déclenchement englobant un élément de percuteur (84) pour pénétrer sélectivement à travers ladite source de gaz sous pression au niveau de ladite partie de paroi pénétrable (82d) au moyen dudit pénétrateur (78), ledit élément de percuteur comportant une rainure à extension axiale (86), recevant une goupille de serrage (88), la goupille de serrage (88) étant configurée de sorte à résister au déplacement dudit élément de percuteur (84) vers ledit pénétrateur (78), et une douille de déclenchement (22) pour déplacer ladite goupille de serrage (88), de sorte à permettre audit élément de percuteur de déplacer ladite source de gaz sous pression sur ledit pénétrateur, ledit assemblage de déclenchement comprenant en outre une goupille de gâchette métallique (94) sur laquelle repose la goupille de serrage (88), ladite douille de déclenchement (22) entourant ledit élément de percuteur (84) et comprenant un bloc de déclenchement (22a) pour déplacer la goupille de serrage (88) par rapport à la goupille de gâchette (94) lors du déplacement axial de ladite douille de déclenchement ; et un moyen (92) pour entraîner l'élément de percuteur à déplacer la source de gaz sous pression sur le pénétrateur.

3. Dispositif d'injection par jet selon les revendications 1 ou 2, dans lequel :
le cylindre à médicament comporte un orifice de sortie (36b) ;
une trajectoire d'écoulement établit une communication entre l'orifice de sortie (36b) et ledit orifice d'injection (18, 418) ;
ledit piston d'injection du médicament (50) coopère, dans une première position, avec le cylindre à médicament pour définir une chambre à volume variable (52) ayant une première taille sélectionnée ;
ladite dose de médicament liquide remplit pratiquement ladite chambre à volume variable (52) en présence d'une première taille sélectionnée, pratiquement sans volume vide ;
ledit piston (50) comporte une deuxième position, coopérant avec ledit cylindre à médicament pour définir une chambre à volume variable (52), ayant une deuxième taille sélectionnée, inférieure à ladite première taille sélectionnée ;
ledit assemblage de pièce à main (12, 112, 412) comporte une première partie de corps (12b, 412b), retenant ladite cartouche d'injection du médicament (14, 114), et un élément de butée (72) pouvant être engagé sélectivement dans ledit piston d'injection du médicament (50), pour déplacer ledit piston d'injection du médicament (50) par l'intermédiaire d'une partie à actionnement par le gaz (60), de ladite première position vers ladite deuxième position ;
et l'éjection de ladite dose de médicament liquide étant entraînée en déplaçant ledit piston d'injection du médicament (50) de ladite deuxième position vers une troisième position.

4. Dispositif selon la revendication 3, dans lequel ledit assemblage de pièce à main (12) englobe en outre un premier alésage (58) dans ladite première partie du corps (12b), ledit piston actionné par le gaz (60) étant reçu de manière mobile dans ledit alésage, et comportant une partie de coulisseau (60e) s'étendant dans ladite cartouche d'injection du médicament (14) pour buter contre ledit piston d'injection du médicament (50), ledit corps et le piston actionné par le gaz coopérant pour définir une première chambre à volume variable à actionnement par le gaz (60d) dans ledit premier alésage (58) ;
ledit assemblage de pièce à main (12) englobant en outre une deuxième partie de corps (12c), s'engageant par ajustement dans ladite première partie du corps (12b), ladite deuxième partie du corps (12c) définissant un deuxième alésage allongé (70), en communication par écoulement du gaz avec ladite première chambre à actionnement par le gaz du premier alésage (60d) et séparé de celle-ci par une partie de paroi, englobant ledit élément de butée (72) supporté par ladite deuxième partie du corps (12c).

5. Dispositif selon la revendication 4, dans lequel ladite première partie du corps (12b) et ladite deuxième partie du corps (12c) sont engagées mutuellement par filetage et ajustement, ladite deuxième partie du corps (12c) supportant ledit élément de butée (72).

6. Dispositif selon la revendication 4, dans lequel ledit deuxième alésage (70) dans ladite deuxième partie du corps (12c) supporte en outre ladite source de gaz sous pression, comprenant une capsule à gaz cylindrique (82).

7. Dispositif selon la revendication 6, dans lequel ladite deuxième partie du corps (12c) dudit assemblage de pièce à main (12) est cylindrique, ladite douille de déclenchement étant une douille de déclenchement tubulaire (22, 122, 412), supportée de manière mobile par ladite deuxième partie du corps (12c) pour entraîner l'ouverture de ladite capsule à gaz (82).

8. Dispositif selon la revendication 3, dans lequel :
ledit cylindre à médicament comporte un élément de bouchon (44) fermant de manière étanche l'orifice de sortie (36b) ;
la chambre à volume variable (52) ayant la deuxième taille sélectionnée est réduite suffisamment par rapport à ladite première taille sélectionnée, de sorte que ledit élément de bouchon (44) est entraîné de manière hydraulique à partir dudit orifice de sortie (36b) ;
ledit assemblage de pièce à main (12) comprend en outre une deuxième partie de corps (12c) établissant ledit élément de butée (72) ;
ladite source de gaz sous pression (82) englobe une capsule à gaz métallique à fermeture étanche (82) ; et
ledit assemblage de déclenchement est configuré de sorte à pénétrer sélectivement dans ladite capsule à gaz (82) et à appliquer une force dudit gaz sous pression audit piston d'injection du médicament (50), pour déplacer ledit piston d'injection du médicament (50) de ladite deuxième position vers ladite troisième position.

9. Dispositif selon la revendication 8, dans lequel ladite première partie du corps (12b) définit un premier alésage (58), ledit piston actionné par le gaz (60) étant reçu de manière mobile dans ledit premier alésage (58), ledit piston actionné par le gaz (60) comportant une tête de piston (60a) et une partie de coulisseau (60e) s'étendant dans ladite cartouche d'injection du médicament (14), pour buter contre ledit piston d'injection du médicament (50), ladite première partie du corps (12b) et ledit piston actionné par le gaz (60) coopérant pour définir une première chambre à volume variable à actionnement par le gaz (60d) dans ledit premier alésage (58) ;
ladite deuxième partie du corps (12c) s'engageant de manière étanche et mobile dans ladite première partie du corps (12b) pour délimiter ladite chambre à actionnement par le gaz (60d), ladite deuxième partie du corps (12c) définissant un deuxième alésage allongé (70), en communication par écoulement de gaz avec ladite chambre à actionnement par le gaz (60d), et ladite capsule à gaz (82) étant reçue dans ledit deuxième alésage (70).

10. Dispositif selon les revendications 1 et 4, dans lequel ledit deuxième alésage dans ladite deuxième partie du corps (412c) supporte en outre ladite source de gaz sous pression, comprenant une capsule à gaz cylindrique (482), reçue de manière mobile dans ledit deuxième alésage, ladite deuxième partie du corps (412c) supportant un capuchon d'extrémité (502) comportant un élément de butée à recul à extension axiale (502a), s'étendant vers ladite capsule à gaz sous pression et entraînant, lors du recul de ladite capsule à gaz sous pression vers ledit capuchon d'extrémité pour limiter le déplacement axial de ladite capsule à gaz sous pression vers ledit capuchon d'extrémité.

11. Dispositif selon la revendication 1, dans lequel :
ladite cartouche d'injection du médicament (414) englobe un orifice de sortie et une trajectoire d'écoulement établissant une communication entre l'orifice de sortie et ledit orifice d'injection (418) ;
un élément de bouchon, agencé dans une première position de manière étanche dans ladite trajectoire d'écoulement ;
ledit piston d'injection du médicament coopérant dans une première position avec ledit cylindre à médicament pour définir une chambre à volume variable (452) ayant une première taille sélectionnée ;
ladite dose de médicament liquide remplissant pratiquement ladite chambre à volume variable (452) en présence de ladite première taille, sans établir pratiquement un volume vide ;
ledit piston d'injection du médicament comportant une deuxième position, coopérant avec ledit cylindre à médicament pour définir une chambre à volume variable (452) ayant une deuxième taille sélectionnée, inférieure à ladite première taille sélectionnée, de sorte que ledit médicament liquide incompressible déplace ledit élément de bouchon de ladite première position, à agencement étanche dans ladite trajectoire d'écoulement, vers une deuxième position, dans laquelle il est bloqué dans ladite trajectoire d'écoulement, ledit cylindre à médicament et ledit élément de bouchon coopérant, dans ladite deuxième position, pour définir une trajectoire d'écoulement ouverte entre ladite chambre à volume variable (452) et ledit orifice d'injection (418) ;
la libération dudit gaz sous pression déplaçant de force ledit piston d'injection du médicament de ladite deuxième position vers une troisième position, de sorte à réduire le volume de ladite chambre à volume variable (452), éjectant ainsi ladite dose de médicament liquide à travers ledit orifice d'injection (418) ; et
ledit assemblage de pièce à main (412) englobant en outre une première partie de corps (412b), retenant ladite cartouche d'injection du médicament (414), et un élément de butée, pouvant être engagé sélectivement dans ledit piston d'injection du médicament par l'intermédiaire d'un piston actionné par le gaz, pour déplacer ledit piston d'injection du médicament de ladite première position vers ladite deuxième position.

12. Dispositif selon la revendication 11, dans lequel ledit assemblage de pièce à main (412) englobe en outre un premier alésage dans ladite première partie du corps (412b), ledit piston d'injection du médicament englobant ledit piston actionné par le gaz (460), reçu de manière mobile dans ledit alésage et comportant une partie de coulisseau s'étendant dans ladite cartouche d'injection du médicament (414), pour buter contre un élément d'étanchéité, qui y est reçu de manière mobile, ledit corps et ledit piston actionné par le gaz (460) coopérant pour définir une première chambre à volume variable à actionnement par le gaz (460d) dans ledit premier alésage ; ledit assemblage de pièce à main (412) englobant en outre une deuxième partie de corps (412c) s'engageant par ajustement dans ladite première partie de corps (412b), ladite deuxième partie de corps (412c) définissant un deuxième alésage allongé, en communication par écoulement de gaz avec ladite chambre à actionnement par le gaz (60d) et séparé de celle-ci par une partie de paroi supportée par ladite deuxième partie de corps (412c).

13. Dispositif selon la revendication 12, dans lequel ledit deuxième alésage dans ladite deuxième partie de corps (412c) supporte en outre une capsule à gaz sous pression cylindrique (482), ladite capsule à gaz cylindrique (182, 482) établissant ladite source de gaz sous pression à fermeture hermétique (182, 482).

14. Dispositif selon la revendication 12, dans lequel lesdites première et deuxième parties du corps sont engagées l'une dans l'autre par filetage, l'une desdites première et deuxième parties de corps englobant une saillie à extension axiale (516), ladite une desdites première et deuxième parties de corps englobant en outre une partie de surface d'extrémité hélicoïdale (518) opposée à l'autre desdites première et deuxième parties de corps, ladite douille de déclenchement (422) à déplacement axial étant bloquée sur ledit corps de la pièce à main, et l'une desdites première et deuxième parties de corps et ladite douille de déclenchement (422) définissant une partie de clavette à extension radiale et axiale (520), l'autre desdites première et deuxième parties de corps et ladite douille de déclenchement (422) définissant une rainure de clavette à extension radiale et axiale (522), dans une première position relative desdites première et deuxième parties de corps, dans laquelle ledit piston d'injection du médicament se trouve dans ladite première position, ladite partie de clavette (520) étant opposée à ladite partie de surface d'extrémité hélicoïdale (518) pour empêcher le déplacement de ladite douille de déclenchement (422), et s'engageant en outre dans ladite saillie (516) pour permettre uniquement une rotation unidirectionnelle relative desdites première et deuxième parties de corps, lesdites parties de corps pouvant effectuer une rotation relative lors de l'engagement fileté dans ladite partie de clavette (520), suivant ladite partie de surface hélicoïdale (518) en vue d'un alignement avec celle-ci et pour permettre une réception dans ladite rainure de clavette dans une deuxième position relative desdites première et deuxième parties de corps, pour déplacer de manière simultanée ledit piston d'injection du médicament de ladite première position vers ladite deuxième position, par l'intermédiaire d'un déplacement axial relatif dudit élément de butée, dans ladite deuxième position desdites première et deuxième parties de corps, ladite douille de déclenchement (422) pouvant être dépalcée pour recevoir ladite partie de clavette (520) dans ladite rainure de clavette (522) et pour entraîner la libération dudit gaz sous pression.

15. Dispositif selon les revendications 1 ou 2, dans lequel :
ledit piston d'injection du médicament peut être déplacé d'une première position vers une deuxième position, ledit piston d'injection du médicament pouvant coopérer avec ladite cartouche d'injection du médicament (14, 414) pour réduire de force le volume dudit cylindre à médicament, en vue de déplacer ledit médicament liquide sous forme d'un jet d'injection à grande vitesse ; et
ledit piston d'injection du médicament (50) étant retenu par un déplacement alternatif par ledit assemblage de pièce à main (12, 412), la libération dudit gaz sous pression déplaçant de force ledit piston d'injection du médicament (50) de manière sélective de ladite première position vers ladite deuxième position ; ledit corps de la pièce à main englobant des première et deuxième parties de corps (12c) engagées mutuellement par filetage, l'une desdites première et deuxième parties de corps (12c) englobant une saillie à extension axiale opposée à l'autre desdites première et deuxième parties de corps (12c), ladite une desdites première et deuxième parties de corps (12c) englobant également une partie de surface d'extrémité hélicoïdale, s'étendant de manière circonférentielle à partir de ladite saillie et étant également opposée à ladite autre desdites première et deuxième surfaces du corps (12c), ladite douille de déclenchement pouvant être déplacée axialement sur ledit corps de la pièce à main, et l'une desdites première et deuxième parties du corps (12c) et ladite douille de déclenchement (22, 122) définissant une partie de clavette à extension radiale et axiale, l'autre desdites première et deuxième parties de corps (12c) et ladite douille de déclenchement (22, 122) définissant une rainure de clavette à extension radiale et axiale, dans une première position relative desdites première et deuxième parties du corps (12c), ladite partie de clavette étant opposée à ladite partie de surface d'extrémité hélicoïdale pour empêcher le déplacement de ladite douille de déclenchement, et s'engageant en outre dans ladite saillie pour permettre uniquement une rotation relative unidirectionnelle desdites première et deuxième parties du corps (12c), lesdites parties du corps pouvant effectuer une rotation relative lors dudit engagement fileté dans ladite partie de clavette, suivant ladite partie de surface hélicoïdale en vue d'un alignement avec celle-ci et pour permettre une réception dans ladite rainure de clavette dans une deuxième position relative desdites première et deuxième parties du corps (12c), dans ladite deuxième position relative desdites première et deuxième parties du corps (12c), ladite douille de déclenchement pouvant être déplacée pour permettre la réception de ladite partie de clavette dans ladite rainure de clavette, pour entraîner la libération dudit gaz sous pression.

16. Dispositif selon la revendication 15, englobant en outre un premier alésage (58) dans ladite première partie du corps (12b), ledit piston d'injection du médicament (50) englobant un piston actionné par le gaz (60), reçu de manière mobile dans ledit alésage et comportant une partie de coulisseau (54), s'étendant dans ladite cartouche d'injection du médicament (14), pour buter contre un élément d'étanchéité qui y est reçu de manière mobile, ledit corps et ledit piston actionné par le gaz (60) coopérant pour définir une première chambre à volume variable à actionnement par le gaz (60d) dans ledit premier alésage (58).

17. Dispositif selon la revendication 1, dans lequel :
ledit assemblage de pièce à main englobe une première partie de corps et une deuxième partie de corps, pouvant tourner l'une par rapport à l'autre ;
l'une desdites première et deuxième parties du corps définissant un doigt d'arrêt (508), comportant une saillie radiale (510) agencée près d'une extrémité distale (508a) de celui-ci, l'autre desdites première et deuxième parties du corps englobant une surface radialement externe, à travers laquelle se déplace ladite extrémité distale (508a) en réponse à la rotation relative desdites parties du corps ; et
dans une première position relative desdites parties du corps, la saillie (510) dudit doigt d'arrêt (508) est reçue dans un évidement (512) défini sur ladite surface radialement externe, et en réponse à la rotation relative desdites parties du corps, ladite extrémité distale dudit doigt d'arrêt (508a) se déplace à travers ladite surface radialement externe, pour faire tomber la saillie (510) radialement dans un deuxième évidement (514) sur ladite surface, la retombée de ladite saillie (510) dans ledit deuxième évidement (514) fournissant une indication visuelle et auditive indiquant que lesdites parties du corps se trouvent dans ladite deuxième position relative.

18. Dispositif selon la revendication 1, dans lequel :
ledit assemblage de pièce à main (412) englobe une première partie de corps (412b) et une deuxième partie de corps (412c) pouvant tourner l'une par rapport à l'autre ;
une partie avant desdites première (412b) et deuxième (412c) parties du corps définit une surface radialement externe à extension axiale, pour permettre une saisie manuelle dudit dispositif, ladite surface présentant une rugosité de surface pour améliorer la saisie manuelle ;
une partie arrière desdites première (412b) et deuxième (412c) parties du corps définit une surface radialement externe, comportant une paire de plateaux diamétralement opposés, à extension radiale, comportant chacun une surface présentant une rugosité pour améliorer la saisie manuelle, en particulier par l'index et le pouce opposés.

19. Dispositif selon la revendication 1, dans lequel la source de gaz sous pression englobe une capsule à gaz (482) pour pénétrer à travers ledit pénétrateur, afin de libérer le gaz sous pression contenu à l'intérieur de ladite capsule à gaz (482) en réponse à l'exécution d'un entraînement axial de ladite capsule à gaz (482) par ledit élément de percuteur (484) sur ledit pénétrateur, ledit élément de percuteur (484) comprenant :
un élément de support de gâchette de type annulaire (484a) ;
plusieurs entretoises de gâchette (490), faisant partie intégrante dudit élément de support de la gâchette (484a) ; et
plusieurs sections d'articulation cassables d'une seule pièce (490a) correspondantes, connectant chacune une entretoise respective desdites plusieurs entretoises de gâchette audit élément de support de la gâchette (484a).

20. Dispositif selon la revendication 1, dans lequel ledit élément de percuteur (184) comprend un élément de percuteur allongé d'une seule pièce moulé à partir de plastique (184), comprenant :
une partie de paroi centrale (218) à extension radiale ;
une partie cylindrique circulaire (216), s'étendant axialement dans une direction à partir de ladite partie de paroi centrale, ladite partie cylindrique circulaire englobant une jupe tubulaire et coopérant avec ladite partie de paroi centrale pour définir un siège de ressort (216a), dans lequel un ressort (192) peut être reçu en vue d'un engagement dans ladite partie de paroi centrale ;
une partie à évasement conique (220a), s'étendant dans une direction axiale opposée à partir de ladite partie de paroi centrale, ladite partie conique englobant plusieurs branches élastiques à espacement circonférentiel (220), s'étendant chacune dans une direction axiale pour se terminer dans une surface d'extrémité axiale respective (220b), pouvant s'engager dans ledit élément de butée de type annulaire (222) dans une première position des branches (220), pour supporter ledit élément de percuteur (184), en s'opposant à la force du ressort (192) exercée sur ladite partie de paroi centrale (218), lesdites branches définissant en coopération, dans ladite première position, un diamètre conique au niveau desdites surfaces d'extrémité, et lesdites branches au niveau desdites surfaces d'extrémité définissant également chacune un rayon circulaire, pratiquement égal au rayon de ladite partie cylindrique circulaire ;
lesdites plusieurs branches (220) pouvant ainsi être déplacées vers une deuxième position, en s'opposant à ladite élasticité desdites branches, lesdites plusieurs branches étant dans ladite deuxième position emboîtées de manière circonférentielle les unes dans les autres et définissant par coopération un diamètre pratiquement égal à celui de ladite partie cylindrique.
